# EUROPEAN PATENT APPLICATION

(11) **EP 2 028 489 A1**
(43) Date of publication of application: **25.02.2009**
(21) Application number: 07016282.1
(22) Date of filing: 20.08.2007
(51) Int. Cl.: G01N 33/50

(54) **Use of AP-2 epsilon**

(71) Applicant: Universität Regensburg, 93053 Regensburg (DE)
(72) Inventor: Bosserhoff, Anja, 93053 Regensburg (DE); Wenke, Ann-Kathrin, 93059 Regensburg (DE)
(74) Representative: Bohmann, Armin K.

(57) **Abstract**

The present invention is related to the use of AP-2 epsilon as a marker for a disease.

SEQUENCE LISTING

<110> Universität Regensburg

<120> Use of AP-2 epsilon

<130> U 10033 EP

<160> 3

<170> PatentIn version 3.3

<210> 1
<211> 434
<212> PRT
<213> Homo sapiens

<220>
<221> mat_peptide
<222> (1)..(434)

<400> 1

Met Glu Arg Pro Asp Gly Leu Gly Ala Ala Ala Gly Gly Ala Arg Leu
1               5                   10                  15

Ser Ser Leu Pro Gln Ala Ala Tyr Gly Pro Ala Pro Pro Leu Cys His
            20                  25                  30

Thr Pro Ala Ala Thr Ala Ala Ala Glu Phe Gln Pro Pro Tyr Phe Pro
        35                  40                  45

Pro Pro Tyr Pro Gln Pro Pro Leu Pro Tyr Gly Gln Ala Pro Asp Ala
    50                  55                  60

Ala Ala Ala Phe Pro His Leu Ala Gly Asp Pro Tyr Gly Gly Leu Ala
65                  70                  75                  80

Pro Leu Ala Gln Pro Gln Pro Pro Gln Ala Ala Trp Ala Ala Pro Arg
                85                  90                  95

Ala Ala Ala Arg Ala His Glu Glu Pro Pro Gly Leu Leu Ala Pro Pro
            100                 105                 110

Ala Arg Ala Leu Gly Leu Asp Pro Arg Arg Asp Tyr Ala Thr Ala Val
        115                 120                 125

Pro Arg Leu Leu His Gly Leu Ala Asp Gly Ala His Gly Leu Ala Asp
    130                 135                 140

Ala Pro Leu Gly Leu Pro Gly Leu Ala Ala Ala Pro Gly Leu Glu Asp
145                 150                 155                 160

Leu Gln Ala Met Asp Glu Pro Gly Met Ser Leu Leu Asp Gln Ser Val
                165                 170                 175

Ile Lys Lys Val Pro Ile Pro Ser Lys Ala Ser Ser Leu Ser Ala Leu
            180                 185                 190

Ser Leu Ala Lys Asp Ser Leu Val Gly Gly Ile Thr Asn Pro Gly Glu
        195                 200                 205

Val Phe Cys Ser Val Pro Gly Arg Leu Ser Leu Leu Ser Ser Thr Ser
    210                 215                 220

Lys Tyr Lys Val Thr Val Gly Glu Val Gln Arg Arg Leu Ser Pro Pro
225                 230                 235                 240

Glu Cys Leu Asn Ala Ser Leu Leu Gly Gly Val Leu Arg Arg Ala Lys
                245                 250                 255

Ser Lys Asn Gly Gly Arg Cys Leu Arg Glu Arg Leu Glu Lys Ile Gly
            260                 265                 270

Leu Asn Leu Pro Ala Gly Arg Arg Lys Ala Ala Asn Val Thr Leu Leu
        275                 280                 285

Thr Ser Leu Val Glu Gly Glu Ala Val His Leu Ala Arg Asp Phe Gly
    290                 295                 300

Tyr Val Cys Glu Thr Glu Phe Pro Ala Lys Ala Ala Ala Glu Tyr Leu
305                 310                 315                 320

Cys Arg Gln His Ala Asp Pro Gly Glu Leu His Ser Arg Lys Ser Met
                325                 330                 335

Leu Leu Ala Ala Lys Gln Ile Cys Lys Glu Phe Ala Asp Leu Met Ala
            340                 345                 350

Gln Asp Arg Ser Pro Leu Gly Asn Ser Arg Pro Ala Leu Ile Leu Glu
        355                 360                 365

Pro Gly Val Gln Ser Cys Leu Thr His Phe Ser Leu Ile Thr His Gly
    370                 375                 380

Phe Gly Gly Pro Ala Ile Cys Ala Ala Leu Thr Ala Phe Gln Asn Tyr
385                 390                 395                 400

Leu Leu Glu Ser Leu Lys Gly Leu Asp Lys Met Phe Leu Ser Ser Val
                405                 410                 415

Gly Ser Gly His Gly Glu Thr Lys Ala Ser Glu Lys Asp Ala Lys His
            420                 425                 430

Arg Lys

<210>  2
<211>  2201
<212>  DNA
<213>  Homo sapiens

<220>
<221>  gene
<222>  (1)..(2201)

<220>
<221>  CDS
<222>  (233)..(1537)

<400>  2
gtgcccgtcc gtcctgcctc catggacccg cccgggaacg gccaccgctg aggaccccac      60

gcccactagg atcccggctg ggtcgcaccc agctaccgca ccgtgacctc cgcgggctgt     120

gccggctccc ggcgcctctg cccgcagcgc tcgccgtcgg gctagggctc cgccgccgcc     180

acgcctcgcg cccggcactc accgccccat gctggtgcac acctactccg cc atg gag     238
                                                          Met Glu
                                                          1

cgc ccc gac ggg ctg gga gca gct gcc ggc ggg gcc cgc ctg tcg tct       286
Arg Pro Asp Gly Leu Gly Ala Ala Ala Gly Gly Ala Arg Leu Ser Ser
        5                   10                  15

ctg ccc cag gcg gcc tac ggg ccg gcg ccc ccg ctc tgc cac acg ccg       334
Leu Pro Gln Ala Ala Tyr Gly Pro Ala Pro Pro Leu Cys His Thr Pro
    20                  25                  30

gcc gcc aca gct gcc gcc gaa ttc cag ccg ccc tac ttc ccg ccg ccc       382
Ala Ala Thr Ala Ala Ala Glu Phe Gln Pro Pro Tyr Phe Pro Pro Pro
35                  40                  45                  50

tac ccg cag cca ccg ctg ccc tac ggt cag gcg ccc gac gcc gcc gca       430
Tyr Pro Gln Pro Pro Leu Pro Tyr Gly Gln Ala Pro Asp Ala Ala Ala
                55                  60                  65

gcc ttt ccc cac ctg gca ggg gac cca tat ggc ggc ctg gcg ccc ctg       478
Ala Phe Pro His Leu Ala Gly Asp Pro Tyr Gly Gly Leu Ala Pro Leu
            70                  75                  80

gcg cag ccg cag cct cct cag gcc gcc tgg gcc gcg ccc cgc gca gcc       526
Ala Gln Pro Gln Pro Pro Gln Ala Ala Trp Ala Ala Pro Arg Ala Ala
        85                  90                  95

gcc cgc gcc cac gag gag cct ccc ggc ctg ctg gca ccg ccc gcc cgc       574
Ala Arg Ala His Glu Glu Pro Pro Gly Leu Leu Ala Pro Pro Ala Arg
    100                 105                 110

gcc ctg ggc ctt gac ccg cgc cgt gac tat gcc act gcc gtg ccc cgg 622
Ala Leu Gly Leu Asp Pro Arg Arg Asp Tyr Ala Thr Ala Val Pro Arg
115                 120                 125                 130

ctc ctg cac ggc ctg gcc gac ggc gcg cac ggc ctg gca gac gca cct 670
Leu Leu His Gly Leu Ala Asp Gly Ala His Gly Leu Ala Asp Ala Pro
                135                 140                 145

ctc ggc ctt ccg ggg ctg gcg gcg gcc ccc ggt ctg gag gac ctg cag 718
Leu Gly Leu Pro Gly Leu Ala Ala Ala Pro Gly Leu Glu Asp Leu Gln
            150                 155                 160

gca atg gac gag ccg gga atg agc ctc cta gac cag tcc gtg atc aag 766
Ala Met Asp Glu Pro Gly Met Ser Leu Leu Asp Gln Ser Val Ile Lys
        165                 170                 175

aaa gtg ccc atc ccc tcc aaa gcc agc agc ctc tca gcc ctc tcc ttg 814
Lys Val Pro Ile Pro Ser Lys Ala Ser Ser Leu Ser Ala Leu Ser Leu
    180                 185                 190

gcc aaa gac agc ctg gtg ggc ggc atc aca aat cct ggt gag gtc ttc 862
Ala Lys Asp Ser Leu Val Gly Gly Ile Thr Asn Pro Gly Glu Val Phe
195                 200                 205                 210

tgc tcc gtg ccc ggc cgg ctt tca ctg ctc agc tca acg tcc aag tac 910
Cys Ser Val Pro Gly Arg Leu Ser Leu Leu Ser Ser Thr Ser Lys Tyr
                215                 220                 225

aag gtg acg gtg ggg gag gtg cag cgg cga ctc tcg cct ccc gag tgc 958
Lys Val Thr Val Gly Glu Val Gln Arg Arg Leu Ser Pro Pro Glu Cys
            230                 235                 240

ctc aac gcc tcc ctc ctg ggg ggt gtc ctc cgc agg gcc aag tcc aaa 1006
Leu Asn Ala Ser Leu Leu Gly Gly Val Leu Arg Arg Ala Lys Ser Lys
        245                 250                 255

aat ggg ggc cgg tgt ttg cgg gaa cgg tta gag aag att ggg ctc aac 1054
Asn Gly Gly Arg Cys Leu Arg Glu Arg Leu Glu Lys Ile Gly Leu Asn
    260                 265                 270

ctg cca gct ggc cgt cgc aag gcc gcc aat gtg acg ctg ctg act tcg 1102
Leu Pro Ala Gly Arg Arg Lys Ala Ala Asn Val Thr Leu Leu Thr Ser
275                 280                 285                 290

cta gtg gaa gga gag gcc gtg cac ctg gcc cga gac ttc ggt tac gtc 1150
Leu Val Glu Gly Glu Ala Val His Leu Ala Arg Asp Phe Gly Tyr Val
                295                 300                 305

tgt gag acg gag ttc cca gcc aag gca gct gcc gag tac ctg tgc cga 1198
Cys Glu Thr Glu Phe Pro Ala Lys Ala Ala Ala Glu Tyr Leu Cys Arg
            310                 315                 320

cag cac gct gac ccg ggg gag ctg cac agc cgc aag agc atg ctg ctg 1246
Gln His Ala Asp Pro Gly Glu Leu His Ser Arg Lys Ser Met Leu Leu
        325                 330                 335

gct gcc aag cag atc tgc aag gag ttt gca gac ttg atg gct cag gac 1294
Ala Ala Lys Gln Ile Cys Lys Glu Phe Ala Asp Leu Met Ala Gln Asp
    340                 345                 350

cgc tca ccg ctg ggc aac agc cgc cca gca ctc atc ctg gag ccc gga 1342
Arg Ser Pro Leu Gly Asn Ser Arg Pro Ala Leu Ile Leu Glu Pro Gly
355                 360                 365                 370

gta cag agc tgc ttg aca cac ttt agc ctc atc acc cat ggc ttc ggt     1390
Val Gln Ser Cys Leu Thr His Phe Ser Leu Ile Thr His Gly Phe Gly
                375                 380                 385

ggg cct gcc atc tgt gct gcc ctc act gcc ttc cag aac tat ttg ctg     1438
Gly Pro Ala Ile Cys Ala Ala Leu Thr Ala Phe Gln Asn Tyr Leu Leu
            390                 395                 400

gag tca ctc aag ggg ctg gac aag atg ttt cta agc agt gtg ggc agt     1486
Glu Ser Leu Lys Gly Leu Asp Lys Met Phe Leu Ser Ser Val Gly Ser
        405                 410                 415

ggg cat ggt gaa acc aag gct tcg gag aag gat gcc aag cat cgg aaa     1534
Gly His Gly Glu Thr Lys Ala Ser Glu Lys Asp Ala Lys His Arg Lys
    420                 425                 430

taa ctgcttctcc caccccatcc ctaaggggct cccaggccct gaaataggga          1587

cttagctctt gggggtgggc ctggaaggac tgaaaggtgg gattagagtc aggccagaaa   1647

gagaacattc atccagagat cccagagttg gggatctggc ttggagtaag ggagggtggc   1707

ctctctgtgg tggtgtgttg gtaagttaag ggcccaggta tttgtctcat gtgtgcaatt   1767

ttctgacctt tgatggttga gaagggtttg gacagaaaat tgacatgaaa agatctggct   1827

catggggcag agccctttcc attagcgtgg ctgggtggcc gtgggtgctt ctagaggcca   1887

aagcctttgt gtttttcact ggtggcagga ggaaaattga taaatcagag gtgctactga   1947

ggagttggtg cccctcattc cagaatctcc tacccccaga aaaggggtgc tggaaggagg   2007

ccccagtgga ctctttgtac ccttcctact ctcagagaga agtgggcagg aggggtcctc   2067

aaggaacaaa gaagataaag cacaaatcgc agaacttgaa tccaggctgc tgctcatcat   2127

agtcctgttg ctgtctgtcc tatttattta tgtatgttgt aattaaattt gaaattttaa   2187

aaaaaaaaaa aaaa                                                     2201

<210>  3
<211>  434
<212>  PRT
<213>  Homo sapiens

<400>  3

Met Glu Arg Pro Asp Gly Leu Gly Ala Ala Ala Gly Gly Ala Arg Leu
1               5                   10                  15

Ser Ser Leu Pro Gln Ala Ala Tyr Gly Pro Ala Pro Pro Leu Cys His
            20                  25                  30

Thr Pro Ala Ala Thr Ala Ala Ala Glu Phe Gln Pro Pro Tyr Phe Pro
        35                  40                  45

Pro Pro Tyr Pro Gln Pro Pro Leu Pro Tyr Gly Gln Ala Pro Asp Ala
    50                  55                  60

Ala Ala Ala Phe Pro His Leu Ala Gly Asp Pro Tyr Gly Gly Leu Ala
65                  70                  75                  80

Pro Leu Ala Gln Pro Gln Pro Pro Gln Ala Ala Trp Ala Ala Pro Arg
                85                  90                  95

Ala Ala Ala Arg Ala His Glu Glu Pro Pro Gly Leu Leu Ala Pro Pro
            100                 105                 110

Ala Arg Ala Leu Gly Leu Asp Pro Arg Arg Asp Tyr Ala Thr Ala Val
        115                 120                 125

Pro Arg Leu Leu His Gly Leu Ala Asp Gly Ala His Gly Leu Ala Asp
    130                 135                 140

Ala Pro Leu Gly Leu Pro Gly Leu Ala Ala Ala Pro Gly Leu Glu Asp
145                 150                 155                 160

Leu Gln Ala Met Asp Glu Pro Gly Met Ser Leu Leu Asp Gln Ser Val
                165                 170                 175

Ile Lys Lys Val Pro Ile Pro Ser Lys Ala Ser Ser Leu Ser Ala Leu
            180                 185                 190

Ser Leu Ala Lys Asp Ser Leu Val Gly Gly Ile Thr Asn Pro Gly Glu
        195                 200                 205

Val Phe Cys Ser Val Pro Gly Arg Leu Ser Leu Leu Ser Ser Thr Ser
    210                 215                 220

Lys Tyr Lys Val Thr Val Gly Glu Val Gln Arg Arg Leu Ser Pro Pro
225                 230                 235                 240

Glu Cys Leu Asn Ala Ser Leu Leu Gly Gly Val Leu Arg Arg Ala Lys
                245                 250                 255

Ser Lys Asn Gly Gly Arg Cys Leu Arg Glu Arg Leu Glu Lys Ile Gly
            260                 265                 270

Leu Asn Leu Pro Ala Gly Arg Arg Lys Ala Ala Asn Val Thr Leu Leu
        275                 280                 285

Thr Ser Leu Val Glu Gly Glu Ala Val His Leu Ala Arg Asp Phe Gly
    290                 295                 300

Tyr Val Cys Glu Thr Glu Phe Pro Ala Lys Ala Ala Ala Glu Tyr Leu

305                 310                 315                 320

Cys Arg Gln His Ala Asp Pro Gly Glu Leu His Ser Arg Lys Ser Met
                325                 330                 335

Leu Leu Ala Ala Lys Gln Ile Cys Lys Glu Phe Ala Asp Leu Met Ala
            340                 345                 350

Gln Asp Arg Ser Pro Leu Gly Asn Ser Arg Pro Ala Leu Ile Leu Glu
        355                 360                 365

Pro Gly Val Gln Ser Cys Leu Thr His Phe Ser Leu Ile Thr His Gly
    370                 375                 380

Phe Gly Gly Pro Ala Ile Cys Ala Ala Leu Thr Ala Phe Gln Asn Tyr
385                 390                 395                 400

Leu Leu Glu Ser Leu Lys Gly Leu Asp Lys Met Phe Leu Ser Ser Val
                405                 410                 415

Gly Ser Gly His Gly Glu Thr Lys Ala Ser Glu Lys Asp Ala Lys His
            420                 425                 430

Arg Lys

## Description

The present invention is related to the use of AP-2 epsilon, the use of interaction partners of AP-2 epsilon and methods for the diagnosis of a disease.

Degenerative chondropathy is a generic term which comprises a number of specific diseases and conditions. Such diseases and conditions are, among others, rheumatoid arthritis, osteo-arthrosis, psoriatic arthritis (PsA), HLA B27-associated oligoarthritis, and relapsing polychondritis.

Rheumatoid arthritis ("RA") is a chronic, inflammatory, systemic disease that produces its most prominent manifestations in affected joints, particularly those of the hands and feet. The onset of rheumatoid arthritis can occur slowly, ranging from a few weeks to a few months, or the condition can surface rapidly in an acute manner.

RA has a worldwide distribution and involves all ethnic groups. Although the disease can occur at any age, the prevalence increases with age and the peak incidence is between the fourth and sixth decade. The prevalence estimates for the North American population vary from 0.3% to 1.5%. Today, over 2,500,000 individuals are diagnosed with rheumatoid arthritis in the United States alone, with some statistics indicating from 6.5 to 8 million potentially afflicted with the disease. Women are affected 2-3 times more often than men.

The early symptoms of rheumatoid arthritis are mostly joint specific such as painful joints with joint swelling or tenderness, but may also include rather non-specific manifestations like stiffness, fever, subcutaneous nodules, and fatigue. Very characteristic is the symmetric involvement of joints. The joints of the hands, feet, knees and wrists are most commonly affected, with eventual involvement of the hips, elbows and shoulders. As the disease progresses, any type of motion becomes very painful and difficult leading eventually to a loss of function of the involved joints. The more severe cases of rheumatoid arthritis can lead to intense pain and joint destruction. Some 300,000 bone and joint replacement surgical procedures are performed annually in an effort to alleviate the pain and mobility loss resultant from arthritis related joint destruction.

The most widely used system to classify RA is the American College of Rheumatology 1987 revised criteria for the classification of RA (Arnett, F.C., et al., Arthritis Rheum. 31 (1988) 315-324). According to these criteria (known as ARA- criteria), a patient is said to have RA if the patient satisfies at least four of the following seven criteria, wherein criteria 1-4 must be present for at least six weeks:
1) morning stiffness for at least one hour,
2) arthritis of three or more joint areas,
3) arthritis of hand joints,
4) symmetrical arthritis,
5) rheumatoid nodules,
6) serum rheumatoid factor ("RF"), and
7) radiographic changes. These criteria have a sensitivity and specificity of approximately 90%.

The only biochemical marker generally accepted (see the above ARA-criteria) and aiding in the diagnosis of RA is the rheumatoid factor (RF) as detected in serum.

The histological changes in RA are not disease-specific but largely depend on the organ involved. The primary inflammatory joint lesion involves the synovium. The earliest changes are injury to the synovial microvasculature with occlusion of the lumen, swelling of endothelial cells, and gaps between endothelial cells, as documented by electron microscopy. This stage is usually associated with mild proliferation of the superficial lining cell layer. Two cell types constitute the synovial lining: bone marrow derived type A synoviocyte, which has macrophage features, and mesenchymal type B synoviocyte. Both cell types contribute to synovial hyperplasia, suggesting a paracrine interaction between these two cell types. This stage of inflammation is associated with congestion, oedema, and fibrin exudation. Cellular infiltration occurs in early disease and initially consists mainly of T lymphocytes. As a consequence of inflammation, the synovium becomes hypertrophic from the proliferation of blood vessels and synovial fibroblasts and from multiplication and enlargement of the synovial lining layers.

Granulation tissue extends to the cartilage and is known as pannus. The tissue actively invades and destroys the periarticular bone and cartilage at the margin between synovium and bone, known as erosive RA.

The articular manifestations of RA can be placed in two categories: reversible signs and symptoms related to inflammatory synovitis and irreversible structural damage caused by synovitis. This concept is useful not only for staging disease and determining prognosis but also for selecting medical or surgical treatment.

Structural damage in the typical patient usually begins sometime between the first and second year of the disease (Van der Heijde, D. M., Br. J. Rheumatol. 34 (1995) 74-78). Although synovitis tends to follow a fluctuating pattern, structural damage progresses as a linear function of the amount of prior synovitis.

The aetiology of the early events in RA remains elusive. An autoimmune component is widely accepted today but other factors are still disputed. The possibility of a bacterial or viral infection has been vigorously pursued. All efforts to associate an infectious agent with RA by isolation, electron microscopy, or molecular biology have failed. It is possible that there is no single primary cause of RA and that different mechanisms may lead to the initial tissue injury and precipitate synovial inflammation.

Clinical signs of synovitis may be subtle and are often subjective. Warm, swollen, obviously inflamed joints are usually seen only in the most active phases of inflammatory synovitis. Cartilage loss and erosion of periarticular bone are the characteristic features of structural damage. The clinical features related to structural damage are marked by progressive deterioration functionally and anatomically. Structural damage to the joint is irreversible and additive.

The effective treatment of rheumatoid arthritis has generally comprised a combination of medication, exercise, rest and proper joint protection therapy. The therapy for a particular patient depends on the severity of the disease and the joints that are involved. Non-steroidal anti-inflammatory drugs, corticosteroids, gold salts, methotrexate and systemic immunosuppressants are widely used to reduce inflammation and joint destruction. The use of steroids and immunosuppressants, however, has significant risks and side effects both in terms of toxicity and vulnerability to potentially lethal conditions. More recently therapeutics based on "biologicals" have been introduced into RA-therapy. Such therapeutics, e.g., are soluble receptors or antibodies directed against TNF-α that significantly reduce inflammation. Though very promising, biologicals are still in limited use due to high costs.

Data from longitudinal clinical and epidemiologic studies provide guidelines for treatment. These studies emphasize 1) the need for early diagnosis, 2) identification of prognostic factors, and 3) early aggressive treatment. Earlier diagnosis and treatment, preferably within the first several months after onset of symptoms, may help prevent irreversible joint damage.

Osteoarthritis or osteoarthrosis (OA), also known as degenerative arthritis, degenerative joint disease, or arthrosis, is a condition in which low-grade inflammation results in pain in the joints, caused by wearing of the cartilage that covers and acts as a cushion inside joints. As the bone surfaces become less well protected by cartilage, the patient experiences pain upon weight bearing, including walking and standing. Due to decreased movement because of the pain, regional muscles may atrophy, and ligaments may become more lax. OA is the most common form of arthritis.

OA affects nearly 21 million people in the United States, accounting for 25% of visits to primary care physicians, and half of all NSAID (Non-Steroidal Anti-Inflammatory Drugs) prescriptions. It is estimated that 80% of the population will have radiographic evidence of OA by age 65, although only 60% of those will be symptomatic. Treatment is with NSAIDs, local injections of glucocorticoid or hyaluronan, and in severe cases, with joint replacement surgery. There has been no cure for OA, as cartilage has not been induced to regenerate.

Primary OA is a chronic degenerative disorder related to but not caused by aging, as there are people well into their nineties who have no clinical or functional signs of the disease. As a person ages, the water content of the cartilage decreases due to a reduced proteoglycan content, thus causing the cartilage to be less resilient. Without the protective effects of the proteoglycans, the collagen fibers of the cartilage can become susceptible to degradation and thus exacerbate the degeneration. Inflammation of the surrounding joint capsule can also occur, though often mild (compared to that which occurs in rheumatoid arthritis). This can happen as breakdown products from the cartilage are released into the synovial space, and the cells lining the joint attempt to remove them. New bone outgrowths, called "spurs" or osteophytes, can form on the margins of the joints, possibly in an attempt to improve the congruence of the articular cartilage surfaces. These bone changes, together with the inflammation, can be both painful and debilitating.

Secondary OA is caused by other factors or diseases but the resulting pathology is the same as for primary OA. These other factors are, e.g. congenital disorders, such as congenital hip luxation, cracking joints, diabetes, inflammatory diseases such as Perthes' disease, Lyme disease, and all chronic forms of arthritis, e.g. costochondritis, gout, and rheumatoid arthritis, injury to joints, as a result of an accident, joint infection, hormonal disorders, ligamentous deterioration or instability, obesity, osteopetrosis, sports injuries, and pregnancy to the joint structures.

Diagnosis is normally done through x-rays. This is possible because loss of cartilage, subchondral ("below cartilage") sclerosis, subchondral cysts, narrowing of the joint space between the articulating bones, and bone spur formation (osteophytes) show up clearly on x-rays. Plain films, however, often do not correlate well with the findings of physical examination of the affected joints.

With or without other techniques, such as MRI (magnetic resonance imaging), arthrocentesis and arthroscopy, diagnosis can be made by a careful study of the duration, location, the character of the joint symptoms, and the appearance of the joints themselves. As yet, there are no methods available to detect OA in its early and potentially treatable stages.

In 1990, the College of Rheumatology, using data from a multi-center study, developed a set of criteria for the of hand osteoarthritis based on hard tissue enlargement and swelling of certain joints. These criteria were found to be 92% sensitive and 98% specific for hand osteoarthritis versus other entities such as rheumatoid arthritis and spondyloarthropities.

Polychondritis is an auto-immune disease in which the human's body's immune system begins to attack and destroy the cartilage tissues in the body.

All cartilage areas can be affected, though in many cases the disease will affect several areas where cartilage is found in the body, and leave others entirely alone. Parts of the body with cartilage, and therefore potentially affected by polychondritis, include the ears, nose, throat, heart valves and of course all areas where musculo-skeletal tissues are connected by cartilage. Specific resultant conditions may include Type 3 Tracheomalacia and Vasculitis.

Reasons for disease onset are not known. Treatment plans typically involve suppression of the immune system with medicines, which often result in a side effect of increasing the risk of other infections.

While the disease can come on at various times, most frequent time for onset is in the late 40's to early 50's. Some literature reports a slightly higher occurrence in females than males, while other literature asserts that sex is apparently not a statistically significant factor in the occurrence rate of the disease. Polychondritis is one of many subclasses of disease in the area of Rheumatology.

It is noteworthy that there is no test that is specific for the diagnosis of polychondritis and more specifically relapsing polychondritis (RPC). The diagnosis is established by the combination of clinical findings, supportive laboratory data, imaging procedures, and biopsy of an involved cartilaginous site. Rather, a spectrum of histologic findings may be present in involved organs. The specific findings depend, in part, on the timing of the biopsy. Two sets of empirically derived diagnostic criteria have been used to define RPC. The original (McAdam's criteria) required the presence of three or more of the following clinical features:
- Bilateral auricular chondritis
- Nonerosive, seronegative inflammatory polyarthritis
- Nasal chondritis
- Ocular inflammation - conjunctivitis, keratitis, scleritis/episcleritis, uveitis
- Respiratory tract chondritis - laryngeal and/or tracheal cartilages; and
- Cochlear and/or vestibular dysfunction - neurosensory hearing loss, tinnitus and/or vertigo

Psoriatic arthritis (PsA) or arthropathic psoriasis is a type of inflammatory arthritis that affects around 5-7% (according to Oxford Clinical Handbook of medicine) of people suffering from the chronic skin condition psoriasis. It occurs more commonly in patients with tissue type HLA-B27. Treatment of psoriatic arthritis is similar to that of rheumatoid arthritis. More than 80% of patients with psoriatic arthritis will have psoriatic nail lesions characterised by pitting of the nails, or more extremely, loss of the nail itself (onycholysis). Psoriatic arthritis is said to be a seronegative spondyloarthropathy.

Psoriatic arthritis can develop at any age, however on average it tends to appear about 10 years after the first signs of psoriasis. For the majority of people this is between the ages of 30 and 50, but it can also affect children. Men and women are equally affected by this condition. In about one in seven cases the arthritis symptoms may occur before any skin involvement.

As well as causing joint inflammation, psoriatic arthritis can cause tendonitis and a sausage-like swelling of the digits known as dactilytis. Radiology will give the appearance of "fluffy, new" bone.

There are still no widely accepted classification or diagnostic criteria for PsA. Currently the descriptive patterns of Moll and Wright are used:
- Distal pattern - the end joints (distal) of the fingers and/or toes
- Oligoarticular pattern - 4 or less joints involved, often in an asymmetric distribution
- Polyarticular pattern - 5 or more joints involved may be symmetrical. This pattern looks like rheumatoid arthritis.
- Spondyloarthritis - involving the spine and sacroiliac joints
- Arthritis mutilans - a severe, deforming form of arthritis

The current aspects of diagnosing PsA are also described, e.g. in P S Helliwell and W J Taylor, Annals of the Rheumatic Diseases 2005;64:ii3-ii8; Classification and diagnostic criteria for psoriatic arthritis.

The problem underlying the present invention is thus to provide a method and means which allow for a reliable diagnosis and staging, respectively, of degenerative chondropathy and the various more specific diseases and conditions, respectively, which are regarded as being manifestations of chondropathy such as osteo-arthrosis, rheumatoid arthritis, psoriatic arthritis (PsA), HLA B27-associated oligoarthritis, and relapsing polychondritis.

A further problem underlying the present invention is to provide a method and means which allow to follow-up the efficacy of a treatment regimen applied to a patient suffering from degenerative chondropathy and one or several of its manifestations.

A still further problem underlying the present invention is to provide a method and means which allow the treatment of degenerative chondropathy and one or severaly of its manifestations.

These and other problems underlying the present invention are solved by the subject matter of the independent claims. Preferred embodiments may be taken from the dependent claims.

The problem underlying the present invention is solved in a first aspect by the use of AP-2 epsilon as a marker for a disease.

In an embodiment the disease is degenerative chondropathy.

In an embodiment the disease is selected from the group comprising osteo-arthrosis, rheumatoid arthritis, psoriatic arthritis (PsA), HLA B27-associated oligoarthritis, and relapsing polychondritis.

In an embodiment the marker is a marker for the course of the disease and/or for therapy monitoring.

In an embodiment the marker is a staging marker for the disease.

In an embodiment the marker is a serum marker or a synovia marker.

In an embodiment AP-2 epsilon comprises an amino acid sequence according to SEQ.ID.No.1.

The problem underlying the present invention is solved in a second aspect by a method for the diagnosis of a disease in a subject, whereby the disease is degenerative chondropathy and the method comprises the step of:
a) providing a sample from the subject; and
b) detecting AP-2 epsilon or a nucleic acid coding therefor.

In an embodiment of the second aspect the method comprises as a further step:
c) comparing the amount of AP-2 epsilon detected in step b) with a reference value, and
d) optionally, determining from the comparing of step c) whether the subject is suffering from degenerative chondropathy or is at risk of suffering therefrom.

In an embodiment of the second aspect the disease is selected from the group comprising osteo-arthrosis and rheumatoid arthritis, psoriatic arthritis (PsA), HLA B27-associated oligoarthritis, and relapsing polychondritis.

In an embodiment of the second aspect the sample is selected from the group comprising serum sample, synovia sample and cartilage sample.

In an embodiment of the second aspect AP-2 epsilon comprises an amino acid sequence according to SEQ.ID.No. 1.

In an embodiment of the second aspect AP-2 epsilon is detected by a means selected from the group comprising sequencing, spectroscopy, MALDI-MS, MALDI-TOF, chromatography, electrophoresis, and interaction with a specific interaction partner, and any combination thereof.

In a preferred embodiment of the second aspect the specific interaction partner is selected from the group comprising antibodies, anticalines, peptide-aptamers, aptamers and spiegelmers.

In an embodiment of the second aspect the nucleic acid coding for AP-2 epsilon comprises a nucleic acid sequence according to SEQ.ID.No.2.

In an embodiment of the second aspect the nucleic acid coding for AP-2 epsilon is detected by a means selected from the group comprising sequencing, hybridization, PCR, spectroscopy, MALDI-MS, MALDI-TOF, chromatography, electrophoresis, and interaction with a specific interaction partner, and any combination thereof.

In a preferred embodiment of the second aspect the specific interaction partner is selected from the group comprising primers and probes.

The problem underlying the present invention is solved in a third aspect by the use of an interaction partner of AP-2 epsilon for the detection of AP-2 epsilon, whereby the interaction partner is selected from the group comprising antibodies, anticalines, peptide-aptamers, aptamers and spiegelmers.

The problem underlying the present invention is solved in a fourth aspect by the use of an interaction partner of the nucleic acid coding for AP-2 epsilon, for the detection of AP-2 epsilon and/or the detection of a nucleic acid coding for AP-2 epsilon, whereby the interaction partner is selected from the group comprising primers and probes.

In an embodiment of the third and fourth aspect the interaction partner is used in a method according to the second aspect.

The problem underlying the present invention is solved in a fifth aspect by a kit for the detection of AP-2 epsilon or a nucleic acid coding therefore, whereby the kit comprises at least a specific interaction partner of AP-2 epsilon or of a nucleic acid coding for AP-2 epsilon, and optionally at least a means selected from the group comprising a reaction vessel, a positive control, a negative control, use instructions for the kit, and auxiliary agent.

The problem underlying the present invention is solved in a sixth aspect by the use of an interaction partner of AP-2 epsilon for the manufacture of a medicament, whereby the interaction partner is selected from the group comprising antibodies, anticalines, peptide-aptamers, aptamers and spiegelmers.

The problem underlying the present invention is solved in a seventh aspect by the use of AP-2 epsilon for the manufacture of a medicament.

In an embodiment of the seventh aspect AP-2 epsilon comprises an amino acid sequence according to SEQ.ID.No.1.

The problem underlying the present invention is solved in an eighth aspect by the use of a nucleic acid coding for AP-2 for the manufacture of a medicament.

In an embodiment of the eighth aspect the nucleic acid codes for AP-2 epsilon having an amino acid sequence according to SEQ.ID.No.1.

In an embodiment of the eighth aspect the nucleic acid comprises
(a) a nucleotide sequence according to SEQ.ID.No. 2, and preferably the complementary strand thereto; or
(b) a nucleotide sequence which, but for the degeneracy of the genetic code, would hybridize to the nucleotide sequence according to SEQ.ID.No. or a nucleotide sequence which is complementary thereto, and preferably the respective complementary strand thereto of each of such sequence.

The present inventors have surprisingly found that AP-2 epsilon is suitable as a marker for a disease, more specifically as a marker for degenerative chondropathy and various manifestation thereof. Additionally, the present inventors have found that AP-2 epsilon is a suitable marker for the course of a disease and/or for monitoring the severity and/or progress of a disease. Still additionally, the present inventors have found that AP-2 epsilon is a synovia marker. These various findings are particularly applicable in case the disease to be diagnosed and assessed, respectively, is degenerative chondropathy and one or several of its manifestations. Without wishing to be bound by any theory, the present inventors assume that AP-2 epsilon is released and/or expressed in chondrocytes upon tissue damage and more specifically cartilage damage such as arising from or in connection with degenerative chondropathy.

AP-2 epsilon is a member of the AP-2 family which comprises highly related transcription factors. The AP-2 transcription factors recognize the palindromic sequence 5'-GCCNNNGGC-3' [1]. AP-2 has been shown to play a crucial role in the control of gene expression in response to cell differentiation signals within neural crest and epidermal cell lineages [2]. So far, five isoforms of the AP-2 gene have been identified from humans and mice that recognize an equal binding motif [3 - 8]. The murine AP-2α gene, which is the only AP-2 isoform reported in connection with cartilage and chondrocytes, respectively, was disrupted by two independent groups by homologous recombination [9, 10]. The AP-2α null mice died perinatally with cranio-abdominoschisis and severe dismorphogenesis of the face and skull, suggesting an AP-2 effect on the skeletal development. Several of the defects associated with the AP-2α-null mutation also affect neural crest cell (NCC) derivatives which includes the craniofacial skeleton, cranial ganglia, and heart outflow tract [11]. A neural crest-specific disruption of the AP-2α gene causes frequent perinatal lethality associated with defects in neural tube closure and cleft secondary palate.

AP-2 epsilon has been described, among others, by Wang et al. (Wang et al., Developmental Dynamics 231: 128-135, 2004). AP-2 epsilon is a polypepitde comprising 434 amino acids and shows the typical modular structure of AP-2 transcription factors with highly conserved C-terminal DNA binding and dimerization domains. AP-2 epsilon has also been described as being involved in the regulation of integrin alpha 10 in chondrocytes (Wenke A-K. et al., Biochemical and Biophysical Research Communications 245 (2006) 495 - 501). However, prior to the present application, the usefulness of AP-2 epsilon as a marker has not been recognized.

As preferably used herein the term AP-2 epsilon includes both the full-length AP-2 epsilon, preferably the one having the amino acid sequence according to SEQ.ID.Nol, and any mutant, fragment and derivative thereof. As preferably used herein a mutant of AP-2 is a polypeptide the amino acid sequence of which differs from the one of wildtype AP-2 epsilon and more specifically from the amino acid sequence according to SEQ.ID.No.1. Such difference may reside in the deletion and/or addition of one or several amino acids. Such deletion and/or addition may occur both at one or both ends of the amino acid sequence or within the amino acid sequence at one or several positions under the proviso that the thus mutated AP-2 exhibits, for example one of the following characteristic, namely being able to bind to the AP-2 DNA binding site, to be detected by AP2 epsilon detecting antibodies, having the conserved structure of AP2 or parts of it (at least in part), at least 30 %, preferably at least 50 and more preferably at least 50 % homology based on the amino acid sequence level.

As preferably used herein a fragment of AP-2 epsilon is a mutant of AP-2 epsilon as described herein, where one or several of the amino acids forming the N terminus and/or one or several of the amino acids forming the C terminus of the polypeptide have been deleted.

As preferably used herein a derivative is AP-2 epsilon, a mutant of AP-2 epsilon or a fragment of AP-2 epsilon, each as described herein, whereby such derivative comprises one or more additional moieties. Preferably such moiety is a label or a targeting agent. In a more preferred embodiment, the label is a radioactive label or a fluorescent label. In another preferred embodiment the targeting agent is an antibody which is at least binding to AP-2 epsilon. In an even more preferred embodiment the antibody is an at least bivalent antibody which is binding with one valence to AP-2 epsilon.

As preferably used herein, a nucleic acid coding for AP-2 epsilon is any nucleic acid coding for AP-2 epsilon, preferably AP-2 epsilon as defined herein. Such nucleic acid also comprises any nucleic acid which but for the degeneracy of the genetic code would hybridize to the nucleic acid coding for AP-2 as described herein and more specifically to the nucleic acid having a nucleic acid sequence according to SEQ.ID.No. 2. In a preferred embodiment such hybridization occurs under stringent conditions as are known to a person skilled in the art. In an embodiment, a marker is an indicator for a condition which allows to link the indicator's presence, or in some cases also its absence, with such condition. Accordingly, a person skilled in the art will be able to determine, from the presence and absence, respectively, of the marker, whether a certain condition is given, realized or existent. It will be understood by the persons skilled in the art that apart from the mere presence and absence, respectively, also the absolute or relative amount of the marker is correlated, in an embodiment, with said condition, its stage and/or its severity.

In an embodiment, the marker of the present invention is a marker for the course of a disease. A course of a disease as preferably used herein is a representation or description of the disease, more specifically its progression or alleviation, over time.

In an embodiment the marker of the present invention is a marker useful for the monitoring of a disease. It will be acknowledged by the persons skilled in the art that it may be essential for the treatment of certain disease to check whether any treatment is effective, and to monitor the course of the disease, more specifically its progression or alleviation, under any treatment regimen.

In a further embodiment, the marker of the present invention may be suitably used as staging marker for a disease. A staging marker as preferably used herein is a marker which allows to determine the stages of a disease, its progression or alleviation. It will be acknowledged by the persons skilled in the art that staging may be crucial for determining an appropriate treatment regimen for a disease, particularly for those diseases where different stages of said disease require different treatment regimens.

In an embodiment, the marker of the present invention is a serum marker or a synovia marker. As preferably used herein, the serum marker is a marker which is contained and/or detected in serum. As preferably used herein a synovia marker is a marker which is contained and/or detected in synovia.

It is within the present invention that the marker of the present invention is detected in a sample. However, it is also within the present invention that the sample is subjected to further processing and the thus processed sample is used for the detection of the marker.

Such sample is preferably a serum sample, a synovia sample or a cartilage sample. As preferably used herein, a serum sample contains at least some serum or is derived from a blood sample. As preferably used herein a synovia sample contains at least some synovia or is derived from synovia. As used herein a cartilage sample contains at least some cartilage or is derived from cartilage tissue.

It is within the present invention that the sample is taken from a subject suffering from a disease or a subject who is suspected of being at risk of suffering from the disease or a subject suffering from the disease without showing clinical symptoms typically associated with such disease. Preferably, the sample is directly taken from the subject. In an embodiment the serum sample is taken from the vasculature of such subject. In an embodiment, the synovia sample is taken from one or more of the joints of such subject. Preferably the joint is one showing degenerative chondropathy or being suspected of developing degenerative chondropathy. In an embodiment, the cartilage sample is taken from one or more of the joints of such subject. Preferably the joint is one showing degenerative chondropathy or being suspected of developing degenerative chondropathy. Methods for retrieving such sample from a subject are known to the persons skilled in the art. In a preferred embodiment retrieving the sample involves the puncture of a vessel by a needle in case of a serum sample, the puncture of a or the joint by a needle in case of a synovia sample and the puncture and removal of cartilage tissue in case of a cartilage sample.

It is within the present invention that the marker of the present invention is detected. The detection of AP-2 epsilon is within the skills of the ordinary person of the art. AP-2 epsilon may be detected as such, i.e. as a polypeptide. Alternatively, AP-2 epsilon may be detected through a nucleic acid coding for AP-2 epsilon. Furthermore, in principle the marker may be detected either directly or indirectly.

Direct detection, either as polypeptide or nucleic acid, can be done by using techniques including, but not limited to sequencing, spectroscopy, MALDI-MS, MALDI-TOF, chromatography, and electrophoresis. Additionally, sequence hybridization and PCR may be used in case a nucleic acid coding for AP-2 epsilon is to be detected. The respective techniques are, for example, described in Lottspeich F./Zorbas H. (eds.), Bioanalytik, Spektrum Akademischer Verlag Heidelberg Berlin, 1998.

Indirect detection involves the detection of AP-2 epsilon or the nucleic acid coding therefore by means of an interaction partner, whereby the interaction partner or the complex consisting of the interaction partner and AP-2 epsilon and the nucleic acid coding therefore, respectively, is either directly or indirectly detected. Methods for the detection of such interaction partners of such complexes are known to the persons skilled in the art. Such methods include, but are not limited to those used for the detection of AP-2 epsilon and the nucleic acid coding therefore, respectively, described herein.

Interaction partners of AP-2 epsilon are known in the art and include, but are not limited to antibodies, anticalines, peptide-aptamers, aptamers and spiegelmers.

Antibodies are preferably used herein comprise both monoclonal antibodies and polyclonal antibodies, the manufacture and generation, respectively, which is well known to the one skilled in the art.

The manufacture of an antibody specific for the protein of AP-2 epsilon or for the nucleic acid coding for AP-2 epsilon, is known to the person skilled in the art and, for example, described in Harlow, E., and Lane, D., "Antibodies: A Laboratory Manual," Cold Spring Harbor Laboratory, Cold Spring Harbor, NY, (1988). Preferably, monoclonal antibodies may be used in connection with the present invention, which may be manufactured according to the protocol of Köhler and Milstein and further developments based thereon. Antibodies as used herein, include, but are not limited to, complete antibodies, antibody fragments or derivatives such as Fab fragments, Fc fragments and single-stranded antibodies, as long as they are suitable and capable of binding to AP-2 epsilon. Apart from monoclonal antibodies also polyclonal antibodies may be used and/or generated. The generation of polyclonal antibodies is also known to the one skilled in the art and, for example, described in Harlow, E., and Lane, D., "Antibodies: A Laboratory Manual," Cold Spring Harbor Laboratory, Cold Spring Harbor, NY, (1988). Preferably, the antibodies used for therapeutic purposes are humanized or human antibodies.

The antibodies, which may be used according to the present invention, may have one or several markers or labels. Such markers or labels may be useful for detecting the antibody either in its diagnostic application or its therapeutic application. Preferably the markers and labels are selected from the group comprising avidin, streptavidin, biotin, gold and fluorescein and used, e. g., in ELISA methods. These and further markers as well as methods are, e. g. described in Harlow, E., and Lane, D., "Antibodies: A Laboratory Manual," Cold Spring Harbor Laboratory, Cold Spring Harbor, NY,(1988). Additionally or alternatively, the antibodies as well as any other AP-2 epsilon antagonist or interaction partner described herein may be a labelled antagonist as more generally described herein.

It is also within the present invention that the label or marker exhibits an additional function apart from detection, such as interaction with other molecules. Such interaction may be, e.g., specific interaction with other compounds. These other compounds may either be those inherent to the system where the antibody is used such as the human or animal body or to the sample which is analysed by using the respective antibody. Appropriate markers may, for example, be biotin or fluoresceine with the specific interaction partners thereof such as avidin and streptavidin and the like being present on the respective compound or structure to interact with the thus marked or labelled antibody. Again this applies also to the other AP-2 epsilon interaction partners described herein.

Another class of interaction partners which can be used in accordance with the present invention are the so-called "anticalines", which are a particular form of target binding polypeptides. Anticalines and their method of manufacture are, among others, described in German patent application DE 197 42 706.

A further class of interaction partner are the so-called peptide-aptamers. Using AP-2 epsilon as a target, peptide aptamers can be generated using a screening process making use of a polypeptide library as described herein in more detail. The selection criterion is that the selected polypeptide is actually binding to AP-2 epsilon.

More specifically, such peptide aptamers may be generated by using methods according to the state of the art such as phage display. Basically, a library of peptide is generated, such as in the form of phages, and this kind of library is contacted with the target molecule, in the present case AP-2 epsilon. Those peptides binding to the target molecule are subsequently removed from the respective reaction, preferably as a complex with the target molecule. It is known to the one skilled in the art that the binding characteristics, at least to a certain extent, depend on the particularly realized experimental set-up such as salt concentration and the like. After separating those polypeptides binding to the target molecule with a higher affinity or a bigger force, from the non-binding members of the library, and optionally also after removal of the target molecule from the complex of target molecule and polypeptide, the respective polypeptide(s) may subsequently be characterised. Prior to the characterisation optionally an amplification step is realized such as, e. g., by propagating the polypeptide coding phages. The characterisation preferably comprises the sequencing of the target binding polypeptides and ultimately of those polypeptides acting as antagonists or interaction partners of AP-2 epsilon as defined herein. Basically, the polypeptides are not limited in their lengths, however, preferably polypeptides having a length from about 8 to 20 amino acids are preferably obtained in the respective methods. The size of the libraries may be about 10² to 10¹⁸, preferably 10⁸ to 10¹⁵ different polypeptides, however, is not limited thereto.

A still further class of interaction partners which can be used in accordance with the present invention are the so-called aptamers. Aptamers are D-nucleic acids which are either single stranded or double stranded and which specifically interact with a target molecule such as AP-2 epsilon. The manufacture or selection of aptamers is, e. g., described in European patent EP 0 533 838. Basically the following steps are realized. First, a mixture of nucleic acids, i. e. potential aptamers, is provided whereby each nucleic acid typically comprises a segment of several, preferably at least eight subsequent randomised nucleotides. This mixture is subsequently contacted with the target molecule, whereby the nucleic acid(s) bind to the target molecule, such as based on an increased affinity towards the target or with a bigger force thereto, compared to the candidate mixture. The binding nucleic acid(s) are/is subsequently separated from the remainder of the mixture. Optionally, the thus obtained nucleic acid(s) is amplified using, e. g. polymerase chain reaction. These steps may be repeated several times giving at the end a mixture of nucleic acids having an increased ratio of nucleic acids specifically binding to the target from which the final binding nucleic acid is then optionally selected. These specifically binding nucleic acid(s) are referred to aptamers. It is obvious that at any stage of the method for the generation or identification of the aptamers samples of the mixture of individual nucleic acids may be taken to determine the sequence thereof using standard techniques. It is within the present invention that the aptamers may be stabilized such as, e. g., by introducing defined chemical groups which are known to the one skilled in the art of generating aptamers. Such modification may for example reside in the introduction of an amino group at the 2'-position of the sugar moiety of the nucleotides. Aptamers are currently used as both therapeutic and diagnostic agents. However, it is also within the present invention that the thus selected or generated aptamers may be used for target validation and/or as lead substance for the development of medicaments, preferably of medicaments based on small molecules. This is actually done by a competition assay whereby the specific interaction between the target molecule and the aptamer is inhibited by a candidate drug whereby upon replacement of the aptamer from the complex of target and aptamer it may be assumed that the respective drug candidate allows a specific inhibition of the interaction between target and aptamer, and if the interaction is specific, said candidate drug will, at least in principle, be suitable to block the target and thus decrease its biological availability or activity in a respective system comprising such target. The thus obtained small molecule may then be subject to further derivatisation and modification to optimise its physical, chemical, biological and/or medical characteristics such as toxicity, specificity, biodegradability and bioavailability. In connection with the present invention, the target is AP-2 epsilon.

A special form of aptamers are Spiegelmers. The generation or manufacture of spiegelmers which may be used or generated according to the present invention using AP-2 epsilon is based on a similar principle. The manufacture of Spiegelmers is described in the international patent application WO 98/08856. Spiegelmers are L-nucleic acids, which means that they are composed of L-nucleotides rather than aptamers which are composed of D-nucleotides as aptamers are. Spiegelmers are characterized by the fact that they have a very high stability in biological system and, comparable to aptamers, specifically interact with the target molecule against which they are directed. In the purpose of generating Spiegelmers, a heterogenous population of D-nucleic acids is created and this population is contacted with the optical antipode of the target molecule, in the present case for example with the D-enantiomer of the naturally occurring L-enantiomer of AP-2 epsilon. Subsequently, those D-nucleic acids are separated which do not interact with the optical antipode of the target molecule. However, those D-nucleic acids interacting with the optical antipode of the target molecule are separated, optionally determined and/or sequenced and subsequently the corresponding L-nucleic acids are synthesized based on the nucleic acid sequence information obtained from the D-nucleic acids. These L-nucleic acids which are identical in terms of sequence with the aforementioned D-nucleic acids interacting with the optical antipode of the target molecule, will specifically interact with the naturally occurring target molecule rather than with the optical antipode thereof. Similar to the method for the generation of aptamers it is also possible to repeat the various steps several times and thus to enrich those nucleic acids specifically interacting with the optical antipode of the target molecule.

It will be acknowledged by the persons skilled in the art that the interaction partners described herein may be generated using a nucleic acid coding for AP-2 epsilon.

The detection of AP-2 epsilon as such is known to or can be perceived by the persons skilled in the art given the disclosure presented herein. In a number of embodiments such detection makes use of the specific interaction between AP-2 epsilon and an interaction partner of AP-2 epsilon, preferably an interaction partner as disclosed herein. Furthermore, it is within the present invention to combine both the use of such interaction partner and the feature of the various AP-2 isoforms, including AP-2 epsilon. Accordingly, in one embodiment, the present invention comprises a method for the detection of AP-2 epsilon, wherein a DNA fragment comprising the DNA binding motif of AP-2, preferably GCCnnnGGC, and more preferably as a double-stranded form, is immobilized to a carrier. Such carrier is contacted with a sample which is to be tested for AP-2 epsilon. If present, AP-2 epsilon will specifically bind to the carrier via the DNA binding motif. As all of the AP-2 isoforms will bind to the DNA binding motif and thus to the carrier carrying such DNA binding motif, a further step is required to discriminate the various AP-2 isoforms. Such further step preferable consists of contacting the AP-2 isoforms bound to the carrier and the carrier, respectively, with an interaction partner specifically binding to or interacting with AP-2 epsilon. Preferably, such interaction partner is an interaction partner according to the present invention or an interaction partner described herein. In an optional further step the interaction partner specifically interacting with AP-2 epsilon is either directly or indirectly detected, preferably by any of the means disclosed herein.

As used herein, a carrier is preferably a solid phase which provides for a surface which allows the immobilization of a molecule, more preferably of a DNA binding motif recognized by AP-2 proteins as preferably defined herein. In an embodiment, the carrier is present as a bead, strip or surface of a flask, preferably a culture flask or a microtitre plate.

In a further aspect the present invention is related to kit for the detection of AP-2 epsilon. Such kit comprises at least one of the interaction partners as disclosed herein and at least one of the following: reaction vessel, buffer, instruction leaflet, positive control, negative control AP-2 epsilon, a nucleic acid coding for AP-2 epsilon. In a preferred embodiment the kit comprises a carrier which comprises a DNA binding motif which allows AP-2 and more particularly AP-2 epsilon to bind to such carrier.

A further group of interaction partners specifically targeting the nucleic acid coding for AP-2 epsilon, including, but not limited to, genomic DNA, cDNA and mRNA, are primers and probes. The design of probes and primers useful in the detection of AP-2 and the nucleic acid coding therefore, are known to the persons skilled in the art and, for example, described in Sambrook, Fritsch, Maniatis: Molecular Cloning, Cold Spring Harbour Laboratory Press 1989. In an embodiment, the primers are used for amplification techniques so as to amplify a nucleic acid coding for AP-2 epsilon. In an embodiment the probes are, for example, used as radioactive probes or for FISH.

Without wishing to be bound by any theory, the present inventors also have reasons to assume that AP-2 epsilon is a therapeutic target, particularly in the treatment of degenerative chondropathy. The involvement of AP-2 in degenerative chondropathy does thus not only allow the diagnosis of such degenerative chondropathy and its various manifestations, particularly those described herein, but also using antagonists and agonists thereof for the treatment and/or prevention of such diseases. It will be acknowledged that depending on the stage of the respective disease either an agonist or an antagonist activity is required so as to provide for a pharmaceutically active agent.

Such antagonists and agonists are described herein in more detail. Such antagonist is in an embodiment an interaction partner of AP-2 epsilon described herein under the proviso that such interaction partner has an antagonist activity to AP-2 epsilon. Furthermore, such agonist is in an embodiment AP-2 epsilon and a derivate thereof as preferably defined herein, or one of the interaction partner of AP-2 epsilon described herein under the proviso that such interaction partner has an agonist activity to AP-2 epsilon. Insofar, the screening methods described herein are also, in principle, suitable for the identification of an agonist for AP-2 epsilon. It will also be understood that the interaction partners to AP-2 epsilon are interaction partners specifically interacting with AP-2 epsilon. Such specific interaction comprises, in a preferred embodiment, the specific binding to, specific replacement of, or specific mimicking of AP-2 epsilon.

Accordingly, particularly the interaction partner of AP-2 epsilon described above may be used in the treatment of degenerative chondropathy and its various manifestations as defined herein, including the use of such interaction partner for the manufacture of a medicament for such purpose. Additionally, so called functional nucleic acid may be used for such purpose.

Said functional nucleic acids are preferably directed against the mRNA of AP-2 epsilon. In principle, using different mode of actions, said functional nucleic acids bind to and degrade the intracellular mRNA of AP-2 epsilon. It is known that such functional nucleic acids have to be transfected or transferred into the chondrocytes. Due to the transitory nature of this kind of functional nucleic acids the expression of AP-2 epsilon is down-regulated for a period of time which can be controlled by, among others, the extent of the transfection of the respective cell and by the stability of the functional nucleic acids. Basically, the knock-down of the AP-2 epsilon shall last until the degenerative process going along with an increased concentration of AP-2 epsilon is returned to a lower level.

It is thus a common feature of all of this functional nucleic acids that they do not interact with the target molecule at the level of the translation product which is in the present case the AP-2 epsilon, but rather interact with the transcription product, i. e. the nucleic acid coding for AP-2 epsilon such as the genomic nucleic acid or any nucleic acid derived therefrom such as the corresponding hnRNA, cDNA and mRNA, respectively. Insofar, the target molecule of the aforementioned classes of compounds is preferably the mRNA of AP-2 epsilon. Examples of such functional nucleic acids are ribozymes, antisense oligonucleotides, and siRNA.

Ribozymes are catalytically active nucleic acids which preferably consist of RNA which basically comprises two moieties. The first moiety shows a catalytic activity, whereas the second moiety is responsible for the specific interaction with the target nucleic acid, in the present case the nucleic acid coding for AP-2 epsilon. Upon interaction between the target nucleic acid and the second moiety of the ribozyme, typically by hybridisation and Watson-Crick base pairing of essentially complementary stretches of bases on the two hybridising strands, the catalytically active moiety may become active which means that it catalyses, either intramolecularly or intermolecularly, the target nucleic acid in case the catalytic activity of the ribozyme is a phosphodiesterase activity. Subsequently, there may be a further degradation of the target nucleic acid which in the end results in the degradation of the target nucleic acid and ultimately reduces the amount of the protein derived from the said target nucleic acid which in the present case is AP-2 epsilon, due to a lack of newly synthesized AP-2 epsilon and a turn-over of prior existing AP-2 epsilon. Ribozymes, their use and design principles are known to the one skilled in the art, and, for example described in Doherty and Doudna (Ribozym structures and mechanism. Annu ref. Biophys. Biomolstruct. 2001 ; 30 :457-75) and Lewin and Hauswirth (Ribozyme Gene Therapy: Applications for molecular medicine. 2001 7: 221-8).

The use of antisense oligonucleotides for the manufacture of a medicament and as a diagnostic agent, respectively, is based on a similar mode of action. Basically, antisense oligonucleotides hybridise based on base complementarity, with a target RNA, preferably with a mRNA, thereby activating RNase H. RNase H is activated by both phosphodiester and phosphorothioate-coupled DNA. Phosphodiester-coupled DNA, however, is rapidly degraded by cellular nucleases with the exception of phosphorothioate-coupled DNA. These resistant, non-naturally occurring DNA derivatives do not inhibit RNase H upon hybridisation with RNA. In other words, antisense polynucleotides are only effective as DNA RNA hybride complexes. Examples for this kind of antisense oligonucleotides are described, among others, in US-patent US 5,849,902 and US 5,989,912. In other words, based on the nucleic acid sequence of the target molecule which in the present case is the nucleic acid coding for AP-2 epsilon, either from the target protein from which a respective nucleic acid sequence may in principle be deduced, or by knowing the nucleic acid sequence as such, particularly the mRNA, suitable antisense oligonucleotides may be designed base on the principle of base complementarity.

Particularly preferred are antisense-oligonucleotides which have a short stretch of phosphorothioate DNA (3 to 9 bases). A minimum of 3 DNA bases is required for activation of bacterial RNase H and a minimum of 5 bases is required for mammalian RNase H activation. In these chimeric oligonucleotides there is a central region that forms a substrate for RNase H that is flanked by hybridising "arms" comprised of modified nucleotides that do not form substrates for RNase H. The hybridising arms of the chimeric oligonucleotides may be modified such as by 2'-O-methyl or 2'-fluoro. Alternative approaches used methylphosphonate or phosphoramidate linkages in said arms. Further embodiments of the antisense oligonucleotide useful in the practice of the present invention are P-methoxyoligonucleotides, partial P-methoxyoligodeoxyribonucleotides or P-methoxyoligonucleotides.

Of particular relevance and usefulness for the present invention are those antisense oligonucleotides as more particularly described in the above two mentioned US patents. These oligonucleotides contain no naturally occurring 5'→3'-linked nucleotides. Rather the oligonucleotides have two types of nucleotides: 2'-deoxyphosphorothioate, which activate RNase H, and 2'-modified nucleotides, which do not. The linkages between the 2'-modified nucleotides can be phosphodiesters, phosphorothioate or P-ethoxyphosphodiester. Activation of RNase H is accomplished by a contiguous RNase H-activating region, which contains between 3 and 5 2'-deoxyphosphorothioate nucleotides to activate bacterial RNase H and between 5 and 10 2'-deoxyphosphorothioate nucleotides to activate eucaryotic and, particularly, mammalian RNase H. Protection from degradation is accomplished by making the 5' and 3' terminal bases highly nuclease resistant and, optionally, by placing a 3' terminal blocking group.

Also an antisense oligonucleotide may be used wherein not the 5' terminal nucleoside is attached to an RNase H-activating region but the 3' terminal nucleoside as specified above. Also, the 5' terminus is selected from the particular group rather than the 3' terminus of said oligonucleotide.

Suitable and useful antisense oligonucleotides are also those comprising a 5' terminal RNase H activating region and having between 5 and 10 contiguous deoxyphosphorothioate nucleotides; between 11 to 59 contiguous 5'→3'-linked 2'-methoxyribonucleotides; and an exonuclease blocking group present at the 3' end of the oligonucleotide that is drawn from the group consisting of a non-5'-3'-phosphodiester-linked nucleotide, from one to three contiguous 5'-3'-linked modified nucleotides and a non-nucleotide chemical blocking group.

Two classes of particularly preferred antisense oligonucleotides can be characterized as follows:

The first class of antisense oligonucleotides comprises a total of 23 nucleotides comprising in 5' → 3' direction a stretch of seven 2'-O-methylribonucleotides, a stretch of nine 2'-deoxyribonucleotides, a stretch of six 2'-O-methylribonucleotides and a 3'-terminal 2'-deoxyribonucleotide. From the first group of seven 2'-O-methylribonucleotides the first four are phosphorothioate linked, whereas the subsequent four 2'-O-methylribonucleotides are phosphodiester linked. Also, there is a phosphodiester linkage between the last, i. e. the most 3'-terminal end of the 2'-O-methylribonucleotides and the first nucleotide of the stretch consisting of nine 2'-deoxyribonucleotides. All of the 2'-deoxyribonucleotides are phosphorothioate linked. A phosphorothioate linkage is also present between the last, i. e. the most 3'-terminal 2'-deoxynucleotide, and the first 2'-O-methylribonucleotide of the subsequent stretch consisting of six 2'-O-methylribonucleotides. From this group of six 2'-O-methylribonucleotides the first four of them, again in 5' → 3' direction, are phosphodiester linked, whereas the last three of them, corresponding to positions 20 to 22 are phosphorothioate linked. The last, i. e. terminal 3'-terminal 2'-deoxynucleotide is linked to the last, i. e. most 3'-terminal 2'-O-methylribonucleotide through a phosphorothioate linkage.

The second class of particularly preferred antisense oligonucleotides also comprises a total of 17 to 23 nucleotides with the following basic structure (in 5' → 3' direction).

At the 5'-terminal end there is an inverted abasic nucleotide which is a structure suitable to confer resistance against exonuclease activity and, e. g., described in WO 99/54459. This inverted abasic is linked to a stretch of five to seven 2'-O-methylribonucleotides which are phosphodiester linked. Following this stretch of five to seven 2'-O-methylribonucleotides there is a stretch of seven to nine 2'-deoxyribonucleotides all of which are phosphorothioate linked. The linkage between the last, i. e. the most 3'-terminal 2'-O-methylribonucleotide and the first 2'-deoxynucleotide of the 2'-deoxynucleotide comprising stretch occurs via a phosphodiester linkage. Adjacent to the stretch of seven to nine 2'-deoxynucleotides a stretch consistent of five to seven 2'-O-methylribonucleotides is connected. The last 2'-deoxynucleotide is linked to the first 2'-O-methylribonucleotide of the latter mentioned stretch consisting of five to seven 2'-O-methylribonucleotides occurs via a phosphorothioate linkage. The stretch of five to seven 2'-O-methylribonucleotides are phosphodiester linked. At the 3'-terminal end of the second stretch of five to seven 2'-O-methylribonucleotide another inverted abasic is attached.

A further class of compounds which are also functional nucleic acids and which may be generated based on the technical teaching given herein and which may be used as medicaments and/or diagnostic agents are small interfering RNA (siRNA) directed to the nucleic acid, preferably mRNA, coding for AP-2 epsilon. siRNA is a double stranded RNA having typically a length of about 19 to about 25 nucleotides. The sequence of one of the two RNA strands corresponds to the sequence of the target nucleic acid such as the nucleic acid coding for AP-2 epsilon, to be degraded. In other words, knowing the nucleic acid sequence of the target molecule, in the present case AP-2 epsilon, preferably the mRNA sequence, a double stranded RNA may be designed with one of the two strands being complementary to said, e. g. mRNA of AP-2 epsilon and, upon application of said siRNA to a system containing the gene, genomic DNA, hnRNA or mRNA coding for AP-2 epsilon, the respective target nucleic acid will be degraded and thus the level of the respective protein be reduced. The basic principles of designing, constructing and using said siRNA as medicament and diagnostic agent, respectively, is, among others, described in international patent applications WO 00/44895 and WO 01/75164.

A further form of functional nucleic acids are microRNAs which are also referred to as miRNAs. miRNAs are single-stranded RNA molecules of about 21-23 nucleotides in length regulating the gene expression. miRNAs are encoded by genes that are transcribed from DNA but not translated into protein and thus consitute non-coding RNA. Instead they are processed from primary transcripts known as *pri-miRNA* to short stem-loop structures called *pre-miRNA* and finally to functional miRNA. Mature miRNA molecules are partially complementary to one or more messenger RNA (mRNA) molecules, and their main function is to downregulate gene expression. They were first described in 1993 by Lee and colleagues (Lee RC, Feinbaum RL, Ambros V (1993). The C. elegans heterochronic gene lin-4 encodes small RNAs with antisense complementarity to lin-14. Cell 75: 843-854), yet the term *microRNA* was only introduced in 2001 in a set of three articles in Science (Ruvkun, G. (Oct 26 2001). "Molecular biology. Glimpses of a tiny RNA world.". Science 294 (5543): 797-9.). The miRNA has the potential to pair to flanking genomic sequences, placing the mature miRNA within an imperfect RNA duplex which may be needed for its processing from a longer precursor transcript. In animals, this processing typically occurs through the action of Drosha and Dicer endonucleases, which excise a miRNA duplex from the hairpin portion of the longer primary transcript. The miRNA duplex comprises the miRNA and a similar-sized segment, known as the miRNA* (miRNA star), from the other arm of the stem-loop. The miRNA is the strand that enters the silencing complex, whereas the miRNA* degrades. Due to this mode of action, miRNA may be used to down-regulate the activity of a nucleic acid coding for AP-2 epsilon. The design of such AP-2 epsilon specific miRNA is obvious to a person of ordinary skills given the disclosure herein.

According to the design principles well known in the art, respective siRNA can be generated. Accordingly, the siRNA claimed herein comprises a stretch of preferably any nucleotide length from 19 to 25 consecutive nucleotides which is either at least partially complementary to the sense or to the antisense strand encoding AP-2 epsilon, and a second ribonucleotide strand which is at least partially complementary to the first one and thus to the antisense strand and sense strand respectively, encoding AP-2 epsilon. Any design principle known in the art of generation or manufacture of siRNA may be applied to this kind of duplex structure. The siRNA space disclosed herein comprises siRNA molecules the antisense strand of which starts with a nucleotides which corresponds to nucleotide no. 1 of an AP-2 epsilon encoding sequence as specified above. Further such siRNA molecules start with a nucleotide which corresponds to nucleotide no 2 of an AP-2 epsilon encoding sequence as specified above, and so on. This kind of scanning over the AP-2 epsilon encoding sequence is repeated so as to provide all possible siRNA molecules which can be directed against AP-2 epsilon. The length of any of the siRNA molecules thus generated may be any length suitable for siRNA, more particularly any length as specified above. Preferably, the various siRNA molecule of the siRNA molecule space disclosed herein, overlap except the most 5' terminal nucleotide of the antisense strand or sense strand. It is obvious that the thus obtained antisense sequences have to complemented through base pairing so as to form the at least partially double-stranded structure required for a functionally active siRNA. It will be understood that the term siRNA as used herein also comprises siNA and RNAi molecules as known to the ones skilled in the art, including any stabilization pattern such as modifying distinct bases in the sequence based on their chemical nature, or modifying the bases forming the siRNA molecules at distinct positions of the sequence. Also, the term siRNA comprises blunt end siRNA molecules as well as molecules having an overhang at the 3'end and/or the 5' end of either the sense-strand or the antisense strand or both.

Based on the aforementioned design principles, it is thus possible to generate such siRNA, antisense oligonucleotide and ribozyme, respectively, once the nucleic acid sequence coding for AP-2 epsilon is known. This is also true for precursor molecules of nucleic acid such as hnRNA, cDNA and the like, including genomic nucleic acid. Of course, also knowing the respective antisense strand may allow the design of such nucleic acid based compounds given the basic principle of base pair complementarity, preferably based on Watson-Crick base pairing.

Based on the mode of action of the aforementioned classes of compounds, such as antibodies, peptides, anticalines, aptamers, spiegelmers, ribozymes, antisense oligonucleotides as well as siRNA, it is thus also within the present invention to use any of these compounds targeting AP-2 epsilon and the nucleic acid coding therefore, respectively, for the manufacture of a medicament or a diagnostic agent for any of the diseases as described herein and any of the diseased conditions described herein. Furthermore, these agents may be used to monitor the progression of said diseases and diseased conditions and the success of any therapy applied, respectively. This applies also to the small molecules the identification of which is also disclosed herein.

As outlined above it is within the present invention that apart from AP-2 epsilon or a part or derivative thereof or a nucleic acid sequence therefore, as described herein, also other means or compounds may be used in order to create or to suppress the effects arising from AP-2 epsilon or the nucleic acid coding for AP-2 epsilon. Put in more general terms, such means may be determined or selected in a screening method. More generally, in such screening method a first step is to provide one or several so called candidate compounds. Candidate compounds as used herein are compounds the suitability of which is to be tested in a test system for treating or alleviating the various diseases as described herein and diseased conditions as described herein or to be used as a diagnostic means or agent for this kind of diseases and diseased conditions. If a candidate compound shows a respective effect in a test system said candidate compound is a suitable means or agent for the treatment of said diseases and diseased conditions and, in principle, as well a suitable diagnostic agent for said diseases and diseased conditions. In a second step the candidate compound is contacted with a AP-2 epsilon expression system or a AP-2 epsilon gene product, preferably a respective gene expression product, such as a hnRNA or mRNA, or a AP-2 epsilon activity system or a AP-2 epsilon. The AP-2 epsilon activity system is also referred to herein as and/or is preferably also active in the meaning of a system detecting the activity of AP-2 epsilon.

A further category of molecules which might be suitable as antagonists to AP-2 epsilon, are small molecules. An aspect of the present invention is thus related to the screening of such molecules using AP-2 epsilon as the target and which preferably is a more preferred embodiment of the above described screening approach.

Insofar, it is within the present invention that the AP-2 epsilon protein as well as the nucleic acid coding for AP-2 epsilon may be used as the target for the manufacture or development of a medicament for the treatment of the diseases described herein and of the diseased conditions described herein, as well as for the manufacture and/or development of means for the diagnosis of said diseases and said conditions, in a screening process, whereby in the screening process small molecules or libraries of small molecules are used. In a preferred embodiment such AP-2 epsilon activity system is an EMSA, i.e. a gel shift assay which confirms the binding of DNA to AP-2. Alternatively, such AP-2 epsilon activity system is a luciferase assay such as described in Wenke at al. (supra). Additionally modulation of AP2 Epsilon can be confirmed by measuring regulation of target genes, preferably as integrin alpha 10. can be used in preferred embodiments. This screening comprises the step of contacting the target molecule with a single small molecule or a variety of small molecules at the same time or subsequently, preferably those from the library as specified above, and identifying those small molecules or members of the library which bind to the target molecules which, if screened in connection with other small molecules may be separated from the non-binding or non-interacting small molecules. It will be acknowledged that the binding and non-binding may strongly be influenced by the particular experimental set-up. In modifying the stringency of the reaction parameters it is possible to vary the degree of binding and non-binding which allows a fine tuning of this screening process. Preferably, after the identification of one or several small molecules which specifically interact with the target molecule, this small molecule may be further characterised. This further characterisation may, for example, reside in the identification of the small molecule and determination of its molecule structure and further physical, chemical, biological and/or medical characteristics. Also preferably, small molecules are those which comply with the Lepinsky rules of five known to the ones skilled in the art. Alternatively, small molecules may also be defined such that they are synthetic-small-molecules, preferably arising from combinatorial chemistry, in contrast to natural products which preferably are non-synthetic. However, it is to be noted that these definitions are only subsidiary to the general understanding of the respective terms in the art.

The screening preferably makes use of an expression system and/or an activity system, respectively. A AP-2 epsilon expression system is basically an expression system which shows or displays the expression of AP-2 epsilon, whereby the extent or level of expression basically may be changed. Preferably, an AP-2 epsilon activity system is essentially an expression system whereby the activity or condition of activity or activation is measured rather than the expression of AP-2 epsilon. In a preferred embodiment such AP-2 epsilon activity system is an EMSA, i.e. a gel shift assay which confirms the binding of DNA to AP-2. Alternatively, such AP-2 epsilon activity system is a luciferase assay such as described in Wenke at al. (supra) assay. In any of these systems it is tested whether under the influence of a candidate compound the activity or activation of AP-2 epsilon or of the nucleic acid coding for AP-2 epsilon is different from the situation without the candidate compound. Regardless whether the particular system is either an expression system or an activity system, it is within the scope of the present invention that either an increase or a decrease of the activity/activation and expression, respectively, may occur and be measured. Typically, the expression system and/or activity/activation system is an in vitro reaction, such as a cell extract or a fraction of the cell extract such as a nucleus extract or a membrane preparation each comprising AP-2 epsilon. An AP-2 epsilon expression system as used herein may also be a cell, preferably a cell of a tissue or organ involved in the diseases as described herein and diseased conditions as described herein. It is within the present invention to use sympathetic cell lines for such purpose.

Whether there is an increase or decrease in the activity system or expression system may be determined at each level of the expression, for example by measuring the increase or decrease of the amount of nucleic acid coding for AP-2 epsilon, more particularly mRNA or the increase or decrease of AP-2 epsilon more particularly of its activity and activation, respectively, expressed or shown under the influence of the candidate compound. The techniques required for the measurement, more particularly the quantitative measurement of this kind of changes, such as for the mRNA or the protein or its activity/activation are known to the one skilled in the art. Also known to the one skilled in the art are methods to determine the amount of or content of AP-2 epsilon, e. g. by the use of appropriate antibodies.

In case of a AP-2 epsilon expression system an increase or decrease of the activity or activation of AP-2 epsilon may be determined, preferably in a functional assay as described above

Contacting the candidate compound and the expression system and activity or activation system, respectively, usually is performed by adding an aqueous solution of the candidate compound to a respective reaction system which is generally referred to herein as test system. Besides aqueous solutions also suspensions or solutions of the candidate compound in organic solvents may be used. The aqueous solution is preferably a buffer solution.

Preferably, in each run using the expression system and activity or activation system, respectively, only a single candidate compound is used. However, it is also within the present invention that several of these kind of tests are performed in parallel in a high throughput system.

A further step in the method according to the present invention resides in determining whether under the influence of the candidate compound the expression or activity of the expression system and activity or activation system, respectively, in relation to AP-2 epsilon or a nucleic acid coding therefore is changed. Typically this is done by comparing the system's reaction upon addition of the candidate compound relative to the one without addition of the candidate compound. Preferably, the candidate compound is a member of a library of compounds.

Basically any library of compounds is suitable for the purpose of this invention regardless of the class of compounds. Suitable libraries of compounds are, among others, libraries composed of small molecules, of peptides, proteins, antibodies, anticalines and functional nucleic acids. The latter compounds may be generated as known to the one skilled in the art and outlined herein. It is also within the present invention that the system described in more detail above in connection with the screening of AP-2 epsilon antagonists based on small molecules, is also applicable to any of the other AP-2 epsilon antagonists and interaction partners, respectively, described herein. It is to be acknowledged that the terms antagonist and interaction partner are preferably used herein in an interchangeable manner, particularly if not indicated to the contrary.

It is also within the present invention that AP-2 epsilon as such and preferably as described herein is used for therapeutic applications such as for the treatment of the various diseases described herein. It is also within the present invention that a nucleic acid coding for AP-2 epsilon is used for therapeutic applications such as for the treatment of the various diseases described herein. It will be acknowledged by the persons skilled in the art that AP-2 epsilon and the nucleic acid coding for AP-2 epsilon, respectively, can be used having either an agonist or an antagonist activity. An agonist activity arises from the presence of AP-2 epsilon, either as a protein or as an expressed nucleic acid coding for AP-2 epsilon, which is suitable to compensate for a lack of AP-2 epsilon activity causing or being associated with the respective disease. An antagonist activity of AP-2 epsilon can, in principle, be created by providing an amount of AP-2 activity which is suitable to antagonize the agonist activity of AP-2 epsilon, preferably the agonist activity of the AP-2 epsilon being present in the diseased subject, organ, and tissue, respectively. In a further embodiment such antagonist activity of AP-2 epsilon can be created by using a truncated form of AP-2 epsilon. Such truncated form of AP-2 epsilon provides for a competition with the full length protein of AP-2 epsilon. Preferably such truncated form of AP-2 epsilon has the feature of a dominant negative form. This can be a variant consisting of the DNA-binding region but lacking the transactivating region or the protein domain important for protein-protein-interaction without the DNA-binding domain and/or the transactivating region of the protein. Lack in DNA or protein-binding can also be achieved by structure or function affecting mutations in the protein-protein, the DNA-binding and/or the transactivating region.

What has been said above in relation to the protein AP-2 epsilon is, in principle also applicable to the nucleic acid coding for AP-2 epsilon. It will be acknowledged by the persons skilled in the art that a nucleic acid coding for AP-2 epsilon may be introduced into a subject, organ, tissue and more specifically a cell forming or being part of such tissue, organ and subject, respectively. More preferably, such nucleic acid is introduced into such cell. In an embodiment the cell has, due to the disease, a decreased or delayed activity of AP-2 epsilon, and such activity is increased or initiated earlier by the introduction of said nucleic acid. It is also within the present invention that the nucleic acid is introduced into a cell showing a normal activity of AP-2 epsilon. It is also within the present invention that the expression of the nucleic acid coding for AP-2 epsilon, preferably the endogenous nucleic acid coding for AP-2 epsilon, is increased or initiated earlier so as to provide for either an agonist or an antagonist activity as outlined above. As preferably used herein, an expression which is initiated earlier is an expression which is initiated at an earlier point in time compared to normal expression, i.e. expression as observed or as would otherwise happen or be observable in the respective cell, which is preferably a diseased cell or a cell affected by the disease, in the course of the disease, without the respective intervention such as by introducing a sequence coding for AP-2 epsilon. As Measures and means are known to the persons skilled in the art on how to practice this. Preferably, the activity of a transcriptional and/or translational regulator, which can be either a positive or a negative regulator, of the nucleic acid coding for AP-2 epsilon is positively or negatively affected resulting in an increased or decreased, or vice versa, transcription and/or translation level or activity of the nucleic acid coding for AP-2 epsilon, preferably of the endogenous nucleic acid coding for AP-2 epsilon.

Methods for the introduction of AP-2, as a protein, into a subject, preferably a diseased subject or a subject presumably being diseased, are known to the persons skilled in the art. Also, methods for the expression of the nucleic acid coding for AP-2 epsilon such as using expression vectors and the like, are known in the art as are methods for introducing such nucleic acid into such kind of cells, and described, e.g. in Maniatis et al (supra).

It will be acknowledged by the ones skilled in the art that the antagonists and agonists, respectively, according to the present invention can, in principle, be locally administered or systemically administered using the various routes known to the ones skilled in the art. However, local administration is preferred, whereby local administration is particularly preferred at diseased joints or diseased cartilage tissue or such sites being suspected of being diseased without clinical symptoms or such sites being at risk to develop such disease.

The various classes of compounds designed according to the present invention such as antibodies, peptides, anticalines, small molecules, aptamers, spiegelmers, ribozymes, antisense oligonucleotides, siRNA and miRNA may also be contained in a pharmaceutical composition. Preferably such pharmaceutical composition is used for the treatment of the diseases as described herein or the diseased conditions described herein. The pharmaceutical composition may comprise in an embodiment one or several of the aforementioned classes of compounds and/or one or more members of a single class, and optionally a further pharmaceutical active compound, and a pharmaceutically acceptable carrier. Such carrier may be either liquid or solid, for example a solution, a buffer, an alcoholic solution or the like. Suitable solid carriers are, among others, starch, liposomes and the like. It is known to the one skilled in the art to provide respective formulations for the various compounds according to the aforementioned classes of compounds in order to realize the particular route of administrations such as oral, parenteral, subcutaneous, intravenous, intramuscular, intraarticular and the like.

The various compounds of the different classes of compounds as mentioned above, may also be, either alone or in combination, subject to or contained in a kit. Such kit comprises apart from the respective compound(s) additionally one or several further elements or compounds whereby the elements are selected from the group comprising buffers, negative controls, positive controls and instructions on the use of the various compounds. Preferably, the various compounds are present in either dry or liquid form, preferably as a unit dosage for a single administration each. The kit may particularly be used for the therapy, diagnosis or monitoring of the progress of the disease or applied therapies in relation to the diseases and diseased conditions as described herein.

The present invention will now be further illustrated by the following figures and examples from which further features, embodiments and advantages may be taken.

### Brief description of the figures.

- Fig. 1: is a diagram showing the extent of mRNA of AP-2 epsilon expression during human mesenchymal stem cell differentiation as determined by means of real-time PCR.
- Figs. 2A and 2B: are diagrams showing the effect of TGFβ-3 on the expression of mRNA of AP-2 epsilon (A), and AP-2 alpha (B) in chondrocytic differentiation of human mesenchymal stem cells as determined by means of quantitative RT-PCR.
- Fig. 3: is a diagram showing the impact of transcriptional regulators Sox-5, Sox-9, AP-2 alpha on the expression of mRNA of AP-2 in chondrocytes as determinded by means of quantitative RT- PCR.
- Figs. 4A, 4B and 4C: are microphotographs of slides immunohistochemically stained for AP-2 epsilon in healthy cartilage (A), in a patient with with rheumatoid arthritis (B) and cartilage from patients with osteoarthritis (C).
- Fig. 5: shows diagrams indicating different expression of mRNA of AP-2 (A), integrin alpha10 (B), AP-2 alpha (C), and Sox-9 (D) in healthy donors and patients with osteoarthritis, as determined by real-time PCR.
- Fig. 6: (A) shows the result of an ELISA assay for AP-2 epsilon performed on serum samples from healthy donors and patients with rheumatoid arthritis, and (B) shows the result of an ELISA assay for AP-2 epsilon performed on synovia samples from patients with rheumatoid arthritis and osteoarthritis.

### Example 1: Materials and Methods

### Cell culture

The chondrosarcoma cell line SW1353 was obtained from the American Type Culture Collection (ATCC#HTB-94). Cells were maintained in DMEM high glucose supplemented with penicillin (400 U/ml), streptomycine (50 µg/ml), L-glutamine (300 µg/ml) and 10% fetal calf serum (FCS; Sigma, Deisenhofen, Germany) and split at a 1:5 ratio every three days. Primary chondrocytes were obtained from Cambrex and cultured as suggested by the manufacturers.

Tissues were obtained from probands or from patients after informed consent following the standards of the Ethics Commission of the University of Regensburg. Normal cartilage with no significant softening or surface fibrillation was obtained at autopsy within 48 h after death. OA cartilage was removed under sterile conditions from femoral condyles of knee joints of patients aged 40-65 years undergoing total knee arthroplasty. Tissue fragments were cut into 1-mm slices and were washed four to five times with Krebs buffer containing 100 units/ml penicillin and 100 µg/ml streptomycin. After two further rinses with calcium-free Dulbecco's modified Eagle's medium (DMEM, Gibco-BRL), the slices were further cut into small pieces and were incubated for 16-24 h with 1.5 mg/ml collagenase B (Roche Diagnostics) and 1 mM cysteine in DMEM. The cell suspension was filtered through nylon gauze (100 µm pore size) and was washed three times with calcium-free DMEM.

Freshly isolated cells were suspended at a density of 1.4 × 10⁶ cells/ml in 1 ml 0.5% low-melting-temperature agarose (FMC, Rockland, ME, USA) in DMEM and were seeded in 35-mm Petri dishes precoated with agarose. The cells were allowed to sediment at 37°C to the interface between the precoating and cell-containing agarose layers. The agarose was allowed to solidify by cooling the cultures to 4°C for 15 min. Further culture conditions were 37°C, 5% CO₂, and 99% humidity. Serum-free DMEM was supplemented with 60 µg/ml β-aminopropionitrile fumarate, 25 µg/ml sodium ascorbate, 1 mM cysteine, 1 mM pyruvate, 100 units/ml penicillin, and 100 µg/ml streptomycin. Culture media were changed every 2-3 days. Conditioned chondrocyte media were collected after 48 h and filtered (0.2 µm) to remove debris.

Human mesenchymal stem cells (HMSC) derived from normal bone marrow (Cell Systems, Katharinen, Germany) were maintained in DMEM supplemented with 50 U/ml penicillin, 50 µg/ml streptomycin, 300 µg/ml 1-glutamine (Invitrogen) and 10% FBS. HMSC differentiation was performed as 3D pellets. For this, 2.5 × 10⁵ HMSCs were seeded into 15 ml polypropylene tubes and pelleted. HMSCs were cultured for 21 days as 3D pellets in serum-free induction medium (DMEM-high glucose, 0.1 µM dexamethasone, 1 mM sodium pyruvate, 0.17 mM ascorbic acid-2-phosphate, 0.35 mM proline and 1:500 insulin-transferrin-selenite (Becton Dickinson) and treated with 10ng/ml TGF-β3 or left untreated (ZITAT).

### Patient sera

Serum was obtained from 50 patients with RA and 10 patients with osteoarthritis (OA) visiting the out-patient clinic of the Division of Rheumatology and Clinical Immunology of the Department of Internal Medicine I of the University of Regensburg. Controls consisted of 50 healthy individuals. Serum samples were aliquoted and stored immediately at -80°C.

### RNA isolation, reverse transcription and PCR

Total cellular RNA was isolated from cultured cells or from tissues using the RNeasy kit (QIAGEN, Hilden, Germany) and cDNAs were generated by reverse transcriptase reaction performed in 20µl reaction volume containing 2 µg of total cellular RNA, 4 µl of 5x first strand buffer (Invitrogen, Groningen, The Netherlands), 2 µl of 0.1 M DTT, 1 µl of dN₆-primer (10mM), 1 µl of dNTPs (10mM) and DEPC-water. The reaction mixture was incubated for 10 min at 70°C, 200 units of Superscript II reverse transcriptase (Invitrogen) were added and RNAs were transcribed for 1 hour at 37°C. Reverse transcriptase was inactivated at 70°C for 10 minutes and the RNA was degraded by digestion with 1 µl RNase A (10 mg/ml) at 37°C for 30 minutes.

PCR analysis for AP-2ε, AP-2a, Sox9 and Integrin alpha10 (ITGA10) were performed using specific primers (*AP*-2ε-for: 5'-GAAATAGGGACTTAGCTCTTGG-3' and -rev: 5'-CCAAGCCAGATCCCCAACTCTG-3' (annealing temperature 59°C), *AP*-2α-for: 5'-GATCCTCGCAGGGACTACA-3' and -rev: 5'-GTTGGACTTGGACAGGGAC-3' (annealing temperature 59°C), *Sox9*-for: 5'-CGAACGCACATCAAGACGA-3' and -rev: 5'-AGGTGAAGGTGGAGTAGAGGC-3' (annealing temperature 58°C), *ITGA10*-for: 5'-CATGAGGTTCACCGCATCACT-3' and *ITGA10*-rev: 5'-AAGGCAAAGGTCACAG TCAAGG-3' (annealing temperature 64°C)). The PCR reaction was performed in a 50 µl reaction volume containing 5 µl 10x Taq-buffer, 1 µl of cDNA, 0,5 µl of each primer (20 mM), 0.5 µl of dNTPs (10 mM), 1 Unit of Taq polymerase and 41,5 µl of water. The amplification reactions were performed by 36 cycles of 1 min at 94°C, 1 min at 58-64°C and a final extension step at 72°C for 1 min. The PCR products were resolved on 1.5% agarose gels. Quantitative real time-PCR was performed with the specific primers and β-actin as housekeeping gene applying Lightcycler technology (Roche, Mannheim, Germany) as described previously (Kuphal et al., 2006).

It will be acknowledged by the persons skilled in the art that the above primers are useful in the detection of AP-2 epsilon and the nucleic acid coding therefore, respectively.

### Immunohistochemical staining

Paraffin sections of cartilage and cartilage isolated from patients with OA or RA were screened for AP-2ε protein expression by immunohistochemistry. The tissues were fixed and subsequently incubated with primary AP-2ε antiserum (1:200) over night at 4°C. The secondary antibody (biotin-labeled anti-rabbit, DAKO, Germany) was incubated for 30 minutes at room temperature, followed by incubation with streptavidin-POD (DAKO) for 30 minutes. Antibody binding was visualized using AEC-solution (DAKO). Finally, the tissues were counterstained by hemalaun solution (DAKO).

### AP-2ε enzyme-linked immunosorbent assay (ELISA)

Two hundred µl of serum from healthy donors and of patients with OA or RA were coated to a well of a 96-well plate (high protein binding, Coming Incorporated, USA) and incubated for two hours at 4°C. Each well was blocked using 100 µl sample diluent No.1 (Bender MedSystems, Vienna, Austria) for 30 minutes at room temperature (RT). The anti-AP-2ε antiserum was used in a 1:750 dilution in sampe diluent. 100 µl were applied to each well and incubated for 60 minutes at RT. After washing for three times with PBS, binding of the polyclonal anti-AP-2 epsilon antibody was detected by a horseradish peroxidase-coupled anti-rabbit IgG antiserum incubated for 30 minutes, then visualized with 200 µl ABTS (Roche) and quantified at O.D. 405 nm after 120 minutes. As a control for specificity BSA was used performing the same kind of assay. Specificity of the antibody was shown in previous studies (Wang et al., Wenke et al).

### Plasmid Constructs

The full-length expression constructs of AP-2ε and AP-2α were cloned into pCMX-PL1 as described previously (Wang et al., Wenke et al.). The full-length Sox5 and Sox9 expression constructs were a gift from Linda Sandell.

### Transient Transfection

DNA transfection of the SW1353 cells was performed using Lipofectamin plus (Invitrogen, Carlsbad, CA). Primary chondrocytes were transfected by electroporation (Amaxa, Cologne, Germany). Briefly, the cells were cultured in 6-well plates. 24 hours after transfection total cellular RNA was isolated using the RNeasy kit (QIAGEN, Hilden, Germany) and cDNA was analyzed. At least three independent transfection experiments were carried out.

### Statistical analysis

Results are expressed as mean ± SD (range) or percent. Comparison between groups was made using the Student's paired t-test. A p value <0.05 was considered statistically significant. All calculations were performed using the GraphPad Prism software (GraphPad software Inc, San Diego, USA).

### Example 2: AP-2 epsilon expression in chondrocytic differentiation

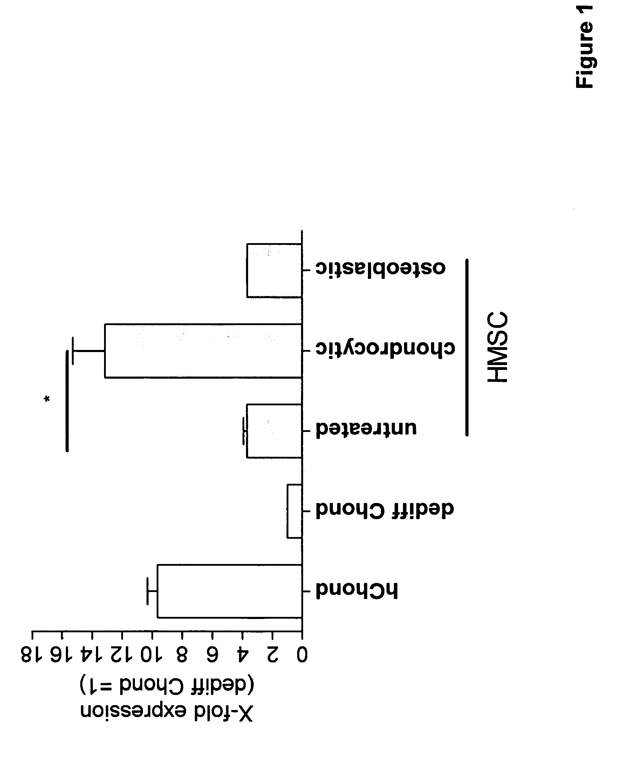

The differentiation process of chondrocytes can be mimicked in cell culture using mesenchymal stem cells (HMSC). We induced chondrocytic differentiation of HMSCs by TGFβ treatment. As a control, mesenchymal stem cells were left untreated or osteoblastic differentiation was induced. Additionally, dedifferentiated chondrocytes and differentiated chondrocytes were analysed for AP-2 epsilon expression. The results are depicted in Fig. 1 which shows AP-2 epsilon expression in human mesenchymal stem cell (HMSC) differentiation. By real-time PCR the amount of AP-2 epsilon mRNA expression was carefully quantified. RNA of differentiated human chondrocytes (hChondro), dedifferentiated chondrocytes (dediff Chondro) and HMSCs either undifferentiated (untreated) or differentiated into chondrocytes or osteoblasts were analyzed.

Expression of AP-2 epsilon was found in differentiated chondrocytes as previously published (Wenke et al.) and in mesenchymal stem cells after chondrocytic differentiation.

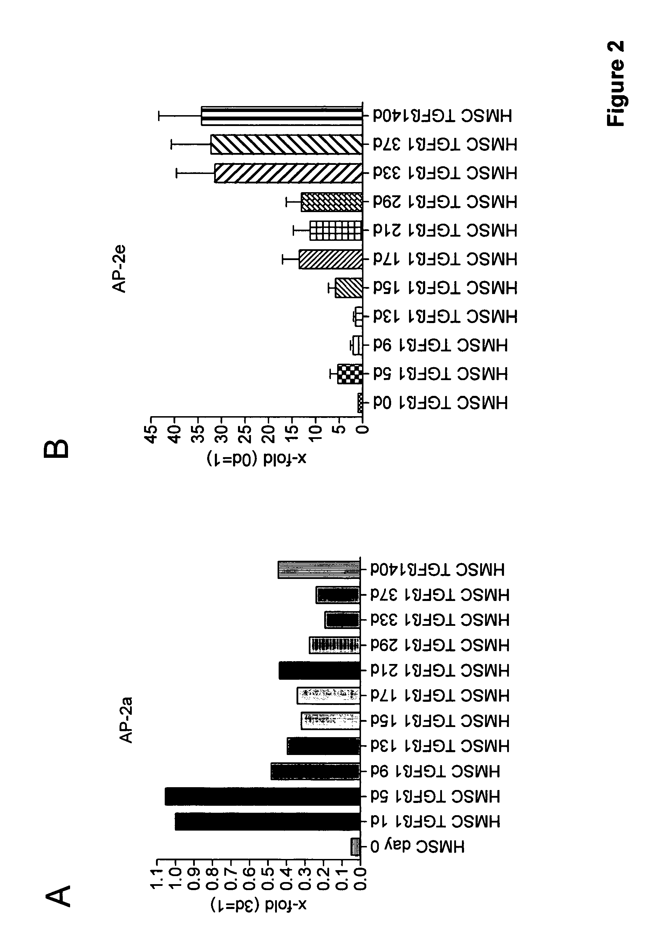

To determine induction of AP-2 epsilon during the differentiation process in detail, differentiation of HMSC into chondrocytes was induced and samples were harvested at defined time points. The results are depicted in Fig.2 which shows mRNA expression of AP-2 epsilon, AP-2 alpha and Sox-9 in HMSC chondrocytic differentiation. Differentiation of HMSC was induced by pellet culture and TGF-β3 and samples were taken every 2 to 8 days following the chondrogenic differentiation until day 40. mRNA expression of AP-2 epsilon (A), and AP-2 alpha (B) was determined by quantitative RT-PCR.

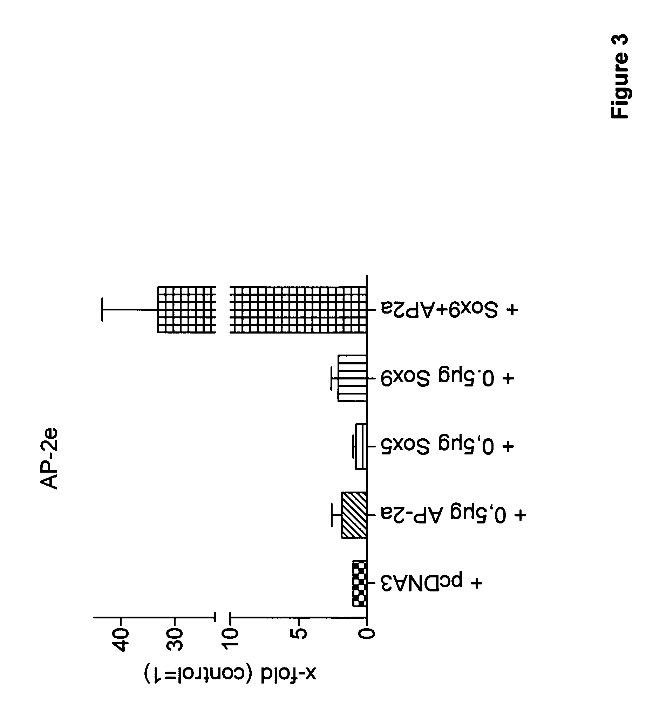

In contrast to AP-2 alpha which was up regulated early in the differentiation process (Fig. 2A), AP-2 epsilon was induced at later time points starting day 17 (Fig. 2B). With regard to these data we were interested whether AP-2 epsilon is regulated by AP-2 alpha and/or Sox-9. Therefore, induction of AP-2 epsilon expression was analyzed in the chondrosarcoma cell line SW1353 transfected with expression plasmids for Sox-9, Sox-5 and AP-2 alpha. The results are depicted in Fig. 3 which shows regulation of AP-2 epsilon expression in chondrocytes. Regulation of AP-2 epsilon by transcriptional regulators (Sox-5, Sox-9, AP-2 alpha) was analyzed by transfection of expression constructs into primary chondrocytes and consecutive analysis of AP-2 epsilon expression by quantitative RT-PCR.

Co-expression of AP-2 alpha and Sox-9 clearly induced AP-2 epsilon expression strongly.

### Example 3: AP-2 epsilon expression in cartilage disease

Expression of AP-2 epsilon in healthy cartilage and cartilage disease such as rheumatoid arthritis and osteoarthritis was determined by immunohistochemistry.

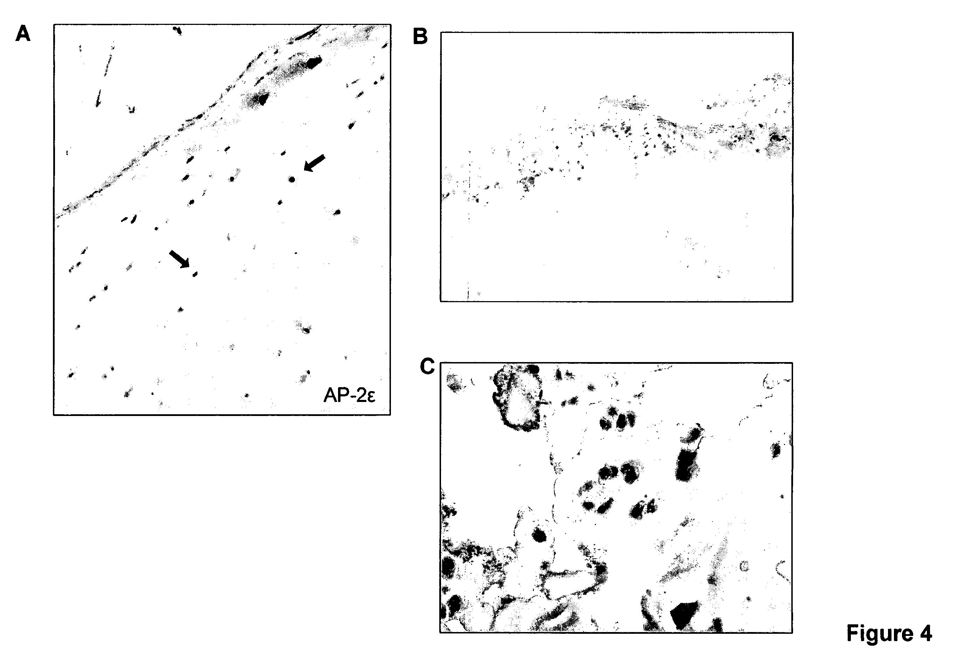

The results are depicted in Fig. 4 which shows immunohistochemical detection of AP-2 epsilon in healthy cartilage and RA and OA samples. AP-2 epsilon protein expression was determined in healthy cartilage (A), cartilage from patients with rheumatoid arthritis (B) and cartilage from patients with osteoarthritis (C) by immunohistochemistry. Chondrocytes expressing AP-2 epsilon are marked by arrows.

Moderate expression of AP-2 epsilon was found in healthy cartilage as already shown previously (Fig. 4A, {Wenke et al.}. In rheumatoid arthritis (Fig. 4B) and osteoarthritis (Fig. 4C) enhanced expression of AP-2 epsilon was revealed.

Due to the unavailability of tissue samples of patients with rheumatoid arthritis we had to concentrate on OA samples and analyzed mRNA expression level compared to cartilage from healthy donors.

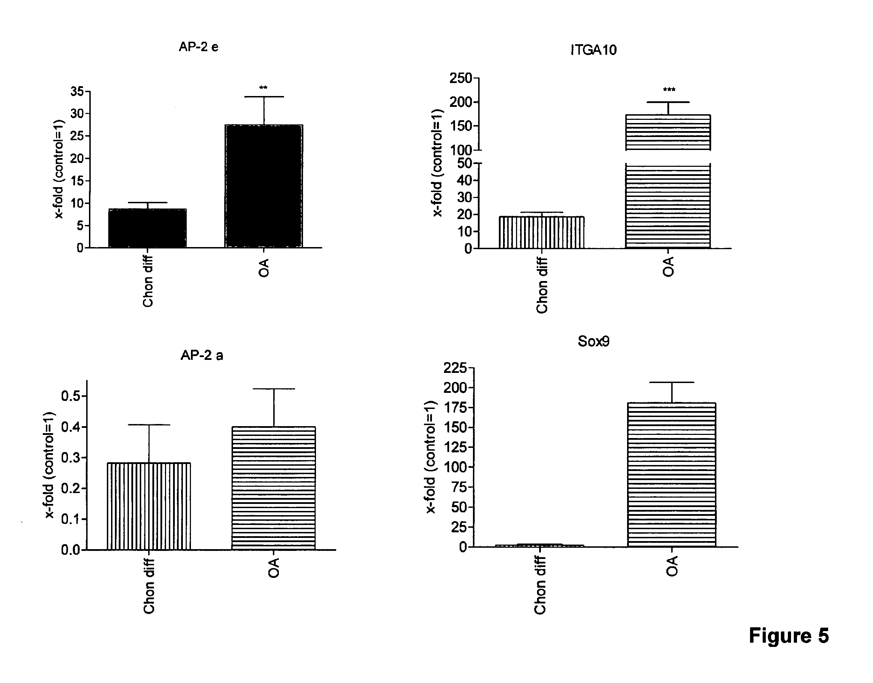

The results are depicted in Fig. 5 which shows expression of AP-2 epsilon RNA expression in OA. By real-time PCR expression of AP-2 epsilon (A), integrin alpha10 (B), AP-2 alpha (C), and Sox-9 (D) mRNA was quantified. RNA was extracted from cartilage of healthy donors (n=3) or patients with OA (n=4). Assays were performed in triplicate. Asterisks indicate P values (p<0.001).

Here, strong induction of AP-2 epsilon was found in OA versus differentiated chondrocytes (Fig. 5A). Consequently, the AP-2 epsilon target genes integrin alpha 10 {Wenke et al.} was also induced (Fig. 5B). In contrast, no change in AP-2 alpha expression was found in OA (Fig. 5C), whereas induction of Sox-9 was determined (Fig. 5D).

Because of these findings we became interested in the regulation of the increase in AP-2 epsilon expression. AP-2 epsilon expression was analyzed in differentiated chondrocytes treated with IL-1. AP-2 epsilon mRNA expression was analyzed in RNA isolated from chondrocytes 24 hours after treatment with 0,5µg IL-1β and from untreated cells.

Here, expression was clearly up regulated by IL-1 compared to untreated controls (not shown).

### Example 4: AP-2 epsilon as marker for RA and OA

As AP-2 epsilon expression was found to be strongly induced in rheumatoid arthritis (RA) and osteoarthritis (OA) and, in general, is restricted to few tissues (mostly neuronal) we aimed to determine whether AP-2 epsilon could serve as a biomarker for RA and OA.

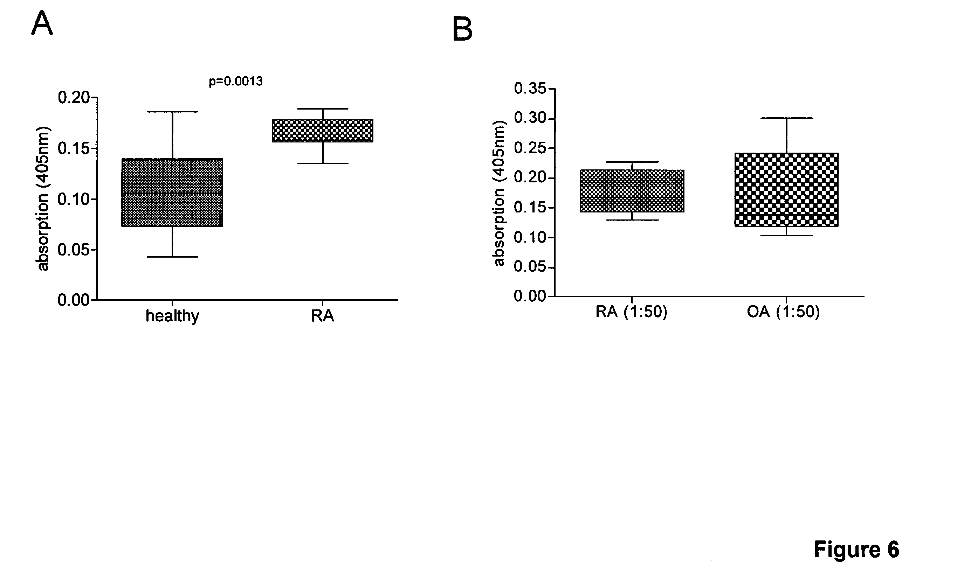

We, therefore, established a one-step ELISA to determine AP-2 epsilon in serum and synovial fluids. Detection of AP-2 epsilon in the ELISA system was inhibitable by the peptide which was used to generate the antibody and by excess of AP-2 epsilon. In 50 samples of sera of RA patients significantly increased levels of AP-2 epsilon were determined compared to healthy controls as depicted in Fig. 6A. For OA only synovia samples were available. Therefore, synovia fluids from OA and RA patients were measured and equal amounts of AP-2 epsilon comparing OA and RA were found (figure 6B).

### References

In connection with the present invention it is referred to the various documents of the prior art as follows, the disclosure of which is incorporated herein by reference.
1. T. Williams and R. Tjian, Analysis of the DNA-binding and activation properties of the human transcription factor AP-2, Genes Dev. 5 (1991) 670-682.
2. P. J. Mitchell, P. M. Timmons, J. M. Hebert, P. W. Rigby, R. Tjian, Transcription factor AP-2 is expressed in neural crest cell lineages during mouse embryogenesis, Genes Dev. 5 (1991) 105-119.
3. M. Oulad-Abdelghani, P. Bouillet, C. Chazaud, P. Dolle, P. Chambon, AP-2.2: a novel AP-2-related transcription factor induced by retinoic acid during differentiation of P19 embryonal carcinoma cells, Exp. Cell Res. 225 (1996) 338-347.
4. M. Moser, A. Imhof, A. Pscherer, R. Bauer, W. Amselgruber, F. Sinowatz, F. Hofstadter, R. Schule, R. Buettner, Cloning and characterization of a second AP-2 transcription factor: AP-2 beta, Development. 121 (1995) 2779-2788.
5. R. Tummala, R. A. Romano, E. Fuchs, S. Sinha, Molecular cloning and characterization of AP-2 epsilon, a fifth member of the AP-2 family, Gene. 321:93-102. (2003) 93-102.
6. H. V. Wang, K. Vaupel, R. Buettner, A. K. Bosserhoff, M. Moser, Identification and embryonic expression of a new AP-2 transcription factor, AP-2 epsilon, Dev. Dyn. 231 (2004) 128-135.
7. F. Zhao, T. Lufkin, B. D. Gelb, Expression of Tfap2d, the gene encoding the transcription factor Ap-2 delta, during mouse embryogenesis, Gene Expr. Patterns 3 (2003) 213-217.
8. U. Werling and H. Schorle, Transcription factor gene AP-2 gamma essential for early murine development, Mol. Cell Biol. 22 (2002) 3149-3156.
9. H. Schorle, P. Meier, M. Buchert, R. Jaenisch, P. J. Mitchell, Transcription factor AP-2 essential for cranial closure and craniofacial development, Nature. 381 (1996) 235-238.
10. J. Zhang, S. Hagopian-Donaldson, G. Serbedzija, J. Elsemore, D. Plehn-Dujowich, A. P. McMahon, R. A. Flavell, T. Williams, Neural tube, skeletal and body wall defects in mice lacking transcription factor AP-2, Nature. 381 (1996) 238-241.
11. S. Brewer, W. Feng, J. Huang, S. Sullivan, T. Williams, Wnt1-Cre-mediated deletion of AP-2alpha causes multiple neural crest-related defects, Dev. Biol. 267 (2004) 135-152.

The features of the present invention disclosed in the specification, the claims and/or the drawings may both separately and in any combination thereof be material for realizing the invention in various forms thereof.

## Claims

1. Use of AP-2 epsilon as a marker for a disease.

2. Use according to claim 1, wherein the disease is degenerative chondropathy.

3. Use according to any of claims 1 to 2, wherein the disease is selected from the group comprising osteo-arthrosis, rheumatoid arthritis, psoriatic arthritis (PsA), HLA B27-associated oligoarthritis, and relapsing polychondritis.

4. Use according to any of claims 1 to 3, wherein the marker is a marker for the course of the disease and/or for therapy monitoring.

5. Use according to any of claims 1 to 4, wherein the marker is a staging marker for the disease.

6. Use according to any of claims 1 to 5, wherein the marker is a serum marker or a synovia marker.

7. Use according to any of claims 1 to 6, wherein AP-2 epsilon comprises an amino acid sequence according to SEQ.ID.No.1.

8. A method for the diagnosis of a disease in a subject, whereby the disease is degenerative chondropathy and the method comprises the step of:
a) providing a sample from the subject; and
b) detecting AP-2 epsilon or a nucleic acid coding therefor.

9. The method according to claim 8, wherein the method comprises as a further step:
c) comparing the amount of AP-2 epsilon detected in step b) with a reference value, and
d) optionally, determining from the comparing of step c) whether the subject is suffering from degenerative chondropathy or is at risk of suffering therefrom.

10. The method according to any of claims 8 to 9, wherein the disease is selected from the group comprising osteo-arthrosis and rheumatoid arthritis, psoriatic arthritis (PsA), HLA B27-associated oligoarthritis, and relapsing polychondritis.

11. The method according to any of claims 8 to 10, wherein the sample is selected from the group comprising serum sample, synovia sample and cartilage sample.

12. The method according to any of claims 8 to 11, wherein AP-2 epsilon comprises an amino acid sequence according to SEQ.ID.No. 1.

13. The method according to any of claims 8 to 11, wherein AP-2 epsilon is detected by a means selected from the group comprising sequencing, spectroscopy, MALDI-MS, MALDI-TOF, chromatography, electrophoresis, and interaction with a specific interaction partner, and any combination thereof.

14. The method according to claim 13, wherein the specific interaction partner is selected from the group comprising antibodies, anticalines, peptide-aptamers, aptamers and spiegelmers.

15. The method according to any of claims 8 to 14, wherein the nucleic acid coding for AP-2 epsilon comprises a nucleic acid sequence according to SEQ.ID.No.2.

16. The method according to any of claims 8 to 15, wherein the nucleic acid coding for AP-2 epsilon is detected by a means selected from the group comprising sequencing, hybridization, PCR, spectroscopy, MALDI-MS, MALDI-TOF, chromatography, electrophoresis, and interaction with a specific interaction partner, and any combination thereof.

17. The method according to claim 16, wherein the specific interaction partner is selected from the group comprising primers and probes.

18. Use of an interaction partner of AP-2 epsilon for the detection of AP-2 epsilon, whereby the interaction partner is selected from the group comprising antibodies, anticalines, peptide-aptamers, aptamers and spiegelmers.

19. Use of an interaction partner of the nucleic acid coding for AP-2 epsilon, for the detection of AP-2 epsilon and/or the detection of a nucleic acid coding for AP-2 epsilon, whereby the interaction partner is selected from the group comprising primers and probes.

20. Use according to any of claims 18 to 19, whereby the interaction partner is used in a method according to any of claims 8 to 17.

21. A kit for the detection of AP-2 epsilon or a nucleic acid coding therefore, whereby the kit comprises at least a specific interaction partner of AP-2 epsilon or of a nucleic acid coding for AP-2 epsilon, and optionally at least a means selected from the group comprising a reaction vessel, a positive control, a negative control, use instructions for the kit, and auxiliary agent.

22. Use of an interaction partner of AP-2 epsilon for the manufacture of a medicament, whereby the interaction partner is selected from the group comprising antibodies, anticalines, peptide-aptamers, aptamers and spiegelmers.

23. Use of AP-2 epsilon for the manufacture of a medicament.

24. Use according to claim 23, wherein AP-2 epsilon comprises an amino acid sequence according to SEQ.ID.No.1.

25. Use of a nucleic acid coding for AP-2 for the manufacture of a medicament.

26. Use according to claim 25, wherein the nucleic acid codes for AP-2 epsilon having an amino acid sequence according to SEQ.ID.No.1.

27. Use according to claim 25 or 26, wherein the nucleic acid comprises
(a) a nucleotide sequence according to SEQ.ID.No. 2, or
(b) a nucleotide sequence which, but for the degeneracy of the genetic code, would hybridize to the nucleotide sequence according to SEQ.ID.No. or a nucleotide sequence which is complementary thereto.
